Europäisches Patentamt

European Patent Office

Office européen des brevets

(11) Publication number: **0 135 291
B1**

# EUROPEAN PATENT SPECIFICATION

(45) Date of publication of patent specification: **14.03.90**

(21) Application number: **84304856.2**

(22) Date of filing: **17.07.84**

(51) Int. Cl.⁵: **C 12 N 15/00, C 12 N 9/12 //
C12R1/19, C12R1/865**

(54) **A modified antibiotic resistance gene.**

(30) Priority: **22.07.83 US 516222
26.09.83 US 535508**

(43) Date of publication of application:
**27.03.85 Bulletin 85/13**

(45) Publication of the grant of the patent:
**14.03.90 Bulletin 90/11**

(84) Designated Contracting States:
**AT BE CH DE FR GB IT LI LU NL SE**

(56) References cited:
**GB-A-2 100 738**

**NATURE, vol. 277, no. 5692, January 11, 1979
(New York, London) A. SINGH et al. "Phenotypic
suppression and misreading in Saccharomyces
cerevisiae" pages 146-148**

(73) Proprietor: **ELI LILLY AND COMPANY
307, East McCarty Street
Indianapolis Indiana 46285 (US)**

(72) Inventor: **Ingolia, Thomas Dominick
1107 Kokomo Lane
Indianapolis Indiana 46241 (US)**
Inventor: **Rao, Ramachandra Nagaraja
6818 Hoover Road
Indianapolis Indiana 46260 (US)**
Inventor: **Kaster, Kevin Ray
8431 Westridge Drive
Indianapolis Indiana 46234 (US)**

(74) Representative: **Hudson, Christopher Mark et al
Erl Wood Manor
Windlesham Surrey GU20 6PH (GB)**

## Description

The present invention relates to a modified hygromycin B resistance-conferring gene. The modified gene is useful for cloning, isolating and characterizing promoters and also for constructing gene fusions that act as dominant selectable markers in appropriate host cells. The invention also relates to vectors and transformants comprising the aforementioned DNA. U.K. Published Patent Application No. 2,100,738 discloses starting materials, including plasmid pKC222 and the hygromycin B resistance-conferring DNA segment therein, which are useful in the present invention. The aforementioned published application does not, however, disclose the modified gene to which the present invention relates or suggest its utility as a critical component of selectable gene fusions.

Gene fusion with a dominant, selectable marker is a useful way to isolate transcriptional or translational activator sequences and thus to express the dominant selectable marker in a foreign system. Since a wide variety of organisms are sensitive to the aminoglycoside antibiotic hygromycin B (Ahmad et al., 1980, Antimicrob. Agents Chemother. 18:789; Mann et al., 1953, Antibiot. and Chemother. 3:1279; Pettinger et al., 1953, Antibiot. and Chemother. 3:1268; and Singh et al., 1979, Nature 277:146), the modified hygromycin B resistance-conferring gene is a valuable dominant selectable marker for use in diverse host systems.

For purposes of the present invention, the last 338, 339 or 340 amino acids of hygromycin B phosphotransferase refers to a polypeptide comprising, in natural sequence, all the amino acids of hygromycin B phosphotransferase except, with reference to the N-terminus of the naturally occurring hygromycin B phosphotransferase molecule, the first, the first and second, or the first, second and third amino acids. In addition, the following terms are as defined below.

Recombinant DNA Cloning Vector — any autonomously replicating agent, including but not limited to plasmids, comprising a DNA molecule to which one or more additional DNA segments can or have been added.

Transformation — the introduction of DNA into a recipient host cell that changes the genotype and results in a change in the recipient cell.

Functional Polypeptide — a recoverable bioactive entirely heterologous polypeptide or precursor, a recoverable bioactive polypeptide comprising a heterologous polypeptide and a portion or whole of a homologous polypeptide, or a recoverable bioinactive fusion polypeptide comprising a heterologous polypeptide and a bio-inactivating homologous polypeptide which can be specifically cleaved.

Fused Gene Product — a recoverable heterologous polypeptide which is fused with a portion or whole of a homologous polypeptide.

Structural Gene — DNA that encodes a functional polypeptide but that lacks transcriptional and translational activator sequences.

The present invention provides a process for preparing a plasmid comprising a DNA sequence encoding the last 338 amino acids of hygromycin B phosphotransferase, either alone or in translational reading phase with a transcriptional and translational activator sequence-containing gene or portion of a gene which comprises ligating the ~1.45 kb EcoRI restriction fragment of pKC222 into EcoRI-digested plasmid pIT122 to obtain plasmid pIT123. The invention further comprises transformants comprising the aforementioned DNA.

More particularly, the DNA of the present invention comprises the deoxyribonucleotide sequence translational activator sequence-containing gene or portion of a gene. The invention further comprises recombinant DNA cloning vectors and transformants comprising the aforementioned DNA.

More particularly, the DNA of the present invention comprises the deoxyribonucleotide sequence

```
                                              Rm
                                              '1
                                              R m
                                              LYS

R²n  CCT GAA CTC ACC GCG ACG TCT GTC GAG AAG TTT CTG
'    ||| ||| ||| ||| ||| ||| ||| ||| ||| ||| ||| |||
R³n  GGA CTT GAG TGG CGC TGC AGA CAG CTC TTC AAA GAC
LYS  PRO GLU LEU THR ALA THR SER VAL GLU LYS PHE LEU

ATC GAA AAG TTC GAC AGC GTC TCC GAC CTG ATG CAG CTC
||| ||| ||| ||| ||| ||| ||| ||| ||| ||| ||| ||| |||
TAG CTT TTC AAG CTG TCG CAG AGG CTG GAC TAC GTC GAG
ILE GLU LYS PHE ASP SER VAL SER ASP LEU MET GLN LEU

TCG GAG GGC GAA GAA TCT CGT GCT TTC AGC TTC GAT GTA
||| ||| ||| ||| ||| ||| ||| ||| ||| ||| ||| ||| |||
AGC CTC CCG CTT CTT AGA GCA CGA AAG TCG AAG CTA CAT
SER GLU GLY GLU GLU SER ARG ALA PHE SER PHE ASP VAL
```

2

```
GGA GGG CGT GGA TAT GTC CTG CGG GTA AAT AGC TGC GCC
!!! !!! !!! !!! !!! !!! !!! !!! !!! !!! !!! !!! !!!
CCT CCC GCA CCT ATA CAG GAC GCC CAT TTA TCG ACG CGG
GLY GLY ARG GLY TYR VAL LEU ARG VAL ASN SER CYS ALA

GAT GGT TTC TAC AAA GAT CGT TAT GTT TAT CGG CAC TTT
!!! !!! !!! !!! !!! !!! !!! !!! !!! !!! !!! !!! !!!
CTA CCA AAG ATG TTT CTA GCA ATA CAA ATA GCC GTG AAA
ASP GLY PHE TYR LYS ASP ARG TYR VAL TYR ARG HIS PHE

GCA TCG GCC GCG CTC CCG ATT CCG GAA GTG CTT GAC ATT
!!! !!! !!! !!! !!! !!! !!! !!! !!! !!! !!! !!! !!!
CGT AGC CGG CGC GAG GGC TAA GGC CTT CAC GAA CTG TAA
ALA SER ALA ALA LEU PRO ILE PRO GLU VAL LEU ASP ILE

GGG GAA TTC AGC GAG AGC CTG ACC TAT TGC ATC TCC CGC
!!! !!! !!! !!! !!! !!! !!! !!! !!! !!! !!! !!! !!!
CCC CTT AAG TCG CTC TCG GAC TGG ATA ACG TAG AGG GCG
GLY GLU PHE SER GLU SER LEU THR TYR CYS ILE SER ARG

CGT GCA CAG GGT GTC ACG TTG CAA GAC CTG CCT GAA ACC
!!! !!! !!! !!! !!! !!! !!! !!! !!! !!! !!! !!! !!!
GCA CGT GTC CCA CAG TGC AAC GTT CTG GAC GGA CTT TGG
ARG ALA GLN GLY VAL THR LEU GLN ASP LEU PRO GLU THR

GAA CTG CCC GCT GTT CTG CAG CCG GTC GCG GAG GCC ATG
!!! !!! !!! !!! !!! !!! !!! !!! !!! !!! !!! !!! !!!
CTT GAC GGG CGA CAA GAC GTC GGC CAG CGC CTC CGG TAC
GLU LEU PRO ALA VAL LEU GLN PRO VAL ALA GLU ALA MET

GAT GCG ATC GCT GCG GCC GAT CTT AGC CAG ACG AGC GGG
!!! !!! !!! !!! !!! !!! !!! !!! !!! !!! !!! !!! !!!
CTA CGC TAG CGA CGC CGG CTA GAA TCG GTC TGC TCG CCC
ASP ALA ILE ALA ALA ALA ASP LEU SER GLN THR SER GLY

TTC GGC CCA TTC GGA CCG CAA GGA ATC GGT CAA TAC ACT
!!! !!! !!! !!! !!! !!! !!! !!! !!! !!! !!! !!! !!!
AAG CCG GGT AAG CCT GGC GTT CCT TAG CCA GTT ATG TGA
PHE GLY PRO PHE GLY PRO GLN GLY ILE GLY GLN TYR THR

ACA TGG CGT GAT TTC ATA TGC GCG ATT GCT GAT CCC CAT
!!! !!! !!! !!! !!! !!! !!! !!! !!! !!! !!! !!! !!!
TGT ACC GCA CTA AAG TAT ACG CGC TAA CGA CTA GGG GTA
THR TRP ARG ASP PHE ILE CYS ALA ILE ALA ASP PRO HIS

GTG TAT CAC TGG CAA ACT GTG ATG GAC GAC ACC GTC AGT
!!! !!! !!! !!! !!! !!! !!! !!! !!! !!! !!! !!! !!!
CAC ATA GTG ACC GTT TGA CAC TAC CTG CTG TGG CAG TCA
VAL TYR HIS TRP GLN THR VAL MET ASP ASP THR VAL SER

GCG TCC GTC GCG CAG GCT CTC GAT GAG CTG ATG CTT TGG
!!! !!! !!! !!! !!! !!! !!! !!! !!! !!! !!! !!! !!!
CGC AGG CAG CGC GTC CGA GAG CTA CTC GAC TAC GAA ACC
ALA SER VAL ALA GLN ALA LEU ASP GLU LEU MET LEU TRP

GCC GAG GAC TGC CCC GAA GTC CGG CAC CTC GTG CAC GCG
!!! !!! !!! !!! !!! !!! !!! !!! !!! !!! !!! !!! !!!
CGG CTC CTG ACG GGG CTT CAG GCC GTG GAG CAC GTG CGC
ALA GLU ASP CYS PRO GLU VAL ARG HIS LEU VAL HIS ALA

GAT TTC GGC TCC AAC AAT GTC CTG ACG GAC AAT GGC CGC
!!! !!! !!! !!! !!! !!! !!! !!! !!! !!! !!! !!! !!!
CTA AAG CCG AGG TTG TTA CAG GAC TGC CTG TTA CCG GCG
ASP PHE GLY SER ASN ASN VAL LEU THR ASP ASN GLY ARG
```

3

```
ATA ACA GCG GTC ATT GAC TGG AGC GAG GCG ATG TTC GGG
 !!! !!! !!! !!! !!! !!! !!! !!! !!! !!! !!! !!! !!!
TAT TGT CGC CAG TAA CTG ACC TCG CTC CGC TAC AAG CCC
ILE THR ALA VAL ILE ASP TRP SER GLU ALA MET PHE GLY

GAT TCC CAA TAC GAG GTC GCC AAC ATC TTC TTC TGG AGG
 !!! !!! !!! !!! !!! !!! !!! !!! !!! !!! !!! !!! !!!
CTA AGG GTT ATG CTC CAG CGG TTG TAG AAG AAG ACC TCC
ASP SER GLN TYR GLU VAL ALA ASN ILE PHE PHE TRP ARG

CCG TGG TTG GCT TGT ATG GAG CAG CAG ACG CGC TAC TTC
 !!! !!! !!! !!! !!! !!! !!! !!! !!! !!! !!! !!! !!!
GGC ACC AAC CGA ACA TAC CTC GTC GTC TGC GCG ATG AAG
PRO TRP LEU ALA CYS MET GLU GLN GLN THR ARG TYR PHE

GAG CGG AGG CAT CCG GAG CTT GCA GGA TCG CCG CGG CTC
 !!! !!! !!! !!! !!! !!! !!! !!! !!! !!! !!! !!! !!!
CTC GCC TCC GTA GGC CTC GAA CGT CCT AGC GGC GCC GAG
GLU ARG ARG HIS PRO GLU LEU ALA GLY SER PRO ARG LEU

CGG GCG TAT ATG CTC CGC ATT GGT CTT GAC CAA CTC TAT
 !!! !!! !!! !!! !!! !!! !!! !!! !!! !!! !!! !!! !!!
GCC CGC ATA TAC GAG GCG TAA CCA GAA CTG GTT GAG ATA
ARG ALA TYR MET LEU ARG ILE GLY LEU ASP GLN LEU TYR

CAG AGC TTG GTT GAC GGC AAT TTC GAT GAT GCA GCT TGG
 !!! !!! !!! !!! !!! !!! !!! !!! !!! !!! !!! !!! !!!
GTC TCG AAC CAA CTG CCG TTA AAG CTA CTA CGT CGA ACC
GLN SER LEU VAL ASP GLY ASN PHE ASP ASP ALA ALA TRP

GCG CAG GGT CGA TGC GAC GCA ATC GTC CGA TCC GGA GCC
 !!! !!! !!! !!! !!! !!! !!! !!! !!! !!! !!! !!! !!!
CGC GTC CCA GCT ACG CTG CGT TAG CAG GCT AGG CCT CGG
ALA GLN GLY ARG CYS ASP ALA ILE VAL ARG SER GLY ALA

GGG ACT GTC GGG CGT ACA CAA ATC GCC CGC AGA AGC GCG
 !!! !!! !!! !!! !!! !!! !!! !!! !!! !!! !!! !!! !!!
CCC TGA CAG CCC GCA TGT GTT TAG CGG GCG TCT TCG CGC
GLY THR VAL GLY ARG THR GLN ILE ALA ARG ARG SER ALA

GCC GTC TGG ACC GAT GGC TGT GTA GAA GTA CTC GCC GAT
 !!! !!! !!! !!! !!! !!! !!! !!! !!! !!! !!! !!! !!!
CGG CAG ACC TGG CTA CCG ACA CAT CTT CAT GAG CGG CTA
ALA VAL TRP THR ASP GLY CYS VAL GLU VAL LEU ALA ASP

AGT GGA AAC CGA CGC CCC AGC ACT CGT CCG AGG GCA AAG
 !!! !!! !!! !!! !!! !!! !!! !!! !!! !!! !!! !!! !!!
TCA CCT TTG GCT GCG GGG TCG TGA GCA GGC TCC CGT TTC
SER GLY ASN ARG ARG PRO SER THR ARG PRO ARG ALA LYS

GAA R⁴
 !!! !5
CTT R⁵
GLU
```

wherein
A is deoxyadenyl,
G is deoxyguanidyl,
C is deoxycytidyl,
T is thymidyl,
R and R² are deoxyribonucleotide triplets that independently encode lysine,
R¹ and R³ are deoxyribonucleotide triplets wherein the nitrogenous bases are complementary to the respective and corresponding bases of R and R²,
m and n = 0 or 1, subject to the limitation that when n = 0, then m = 0 and when m = 1, then n = 1,
R⁴ is a deoxyribonucleotide triplet that encodes a translational stop codon and
R⁵ is a deoxyribonucleotide triplet wherein the nitrogenous bases are complementary to the corresponding bases of R⁴.

The amino acids encoded by the above DNA are designated below the appropriate nucleotide triplet. Accordingly,

MET is methionine,
LYS is lysine,
PRO is proline,
GLU is glutamic acid,
LEU is leucine,
THR is threonine,
ALA is alanine,
SER is serine,
VAL is valine,
PHE is phenylalanine,
ILE is isoleucine,
GLY is glycine,
ASP is aspartic acid,
GLN is glutamine,
ARG is arginine,
CYS is cysteine,
TRP is tryptophan,
ASN is asparagine,
HIS is histidine and
TYR is tyrosine.

The present invention, of which R, $R^1$, $R^2$, $R^3$, $R^4$ and $R^5$ are defined in accordance with the genetic code (Watson, J. D., 1976, Molecular Biology of the Gene, W. A. Benjamin Inc., Menlo Park, California), can be conventionally synthesized by the modified phosphotriester method using fully protected trideoxyribonucleotide building blocks. Such synthetic methods are well known in the art and can be carried out in substantial accordance with the procedure of Itakura *et al.,* 1977, Science 198:1056 and Crea *et al.,* 1978, Proc. Nat. Acad. Sci. USA 75:5765. Those skilled in the art will recognize that other DNA sequences encoding the same amino acids as those encoded by the above illustrative DNA sequence can also be synthesized. These other DNA sequences reflect the degeneracy of the genetic code and thus are within the scope of the present invention.

The above-defined DNA wherein m = 0, $R^4$ is TAG and $R^5$ is ATC can also be constructed by appropriate digestion of plasmid pKC222. The convenient *Hph*I restriction site near the beginning of the coding region of the hygromycin B phosphotransferase gene in pKC222 is very useful for this purpose. Thus, *Hph*I-*Pst*I digestion of plasmid pKC222 results in a truncated hygromycin B phosphotransferase gene which comprises 325 bp (plus single stranded extensions) and which encodes amino acids 4—112 of the hygromycin B phosphotransferase polypeptide. After removal of the 3′ extension left by the *Hph*I restriction enzyme, the fragment can be provided with a *Bam*HI molecular linker, digested with *Eco*RI restriction enzyme and then ligated to *Bam*HI-*Eco*RI-digested plasmid pBR322. The resultant plasmid, designated as pIT122, contains only part of the hygromycin B phosphotransferase gene and is used as a starting material.

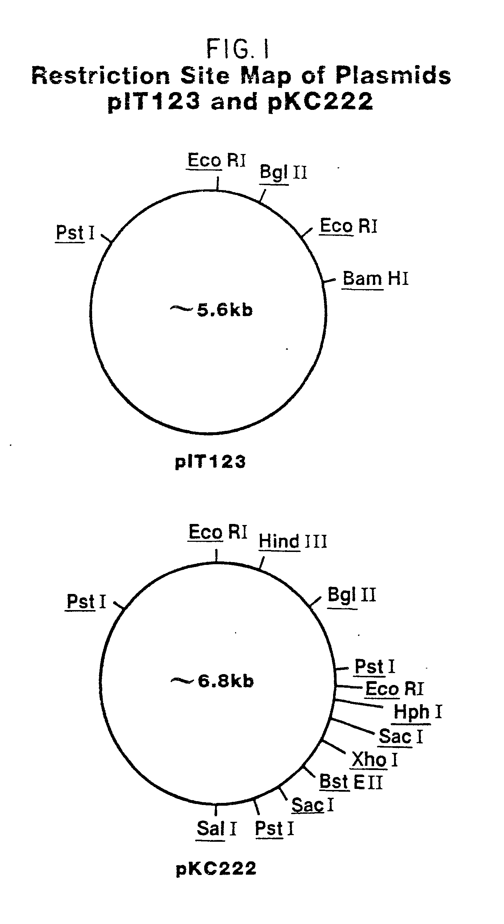

Coding information for hygromycin B phosphotransferase amino acids 113—341 can be provided by ligating the ~1.45 kb *Eco*RI fragment of plasmid pKC222 into appropriately cleaved plasmid pIT122. The resultant plasmid, designated as pIT123, contains the complete hygromycin B phosphotransferase structural gene except for the substitution of the *Bam*HI linker for the first 9 nucleotide pairs. The truncated gene thus encodes a hygromycin B phosphotransferase that lacks the first 3 amino acids encoded by the native gene. A restriction site map of plasmid pIT123 is presented in Figure 1 of the accompanying drawings.

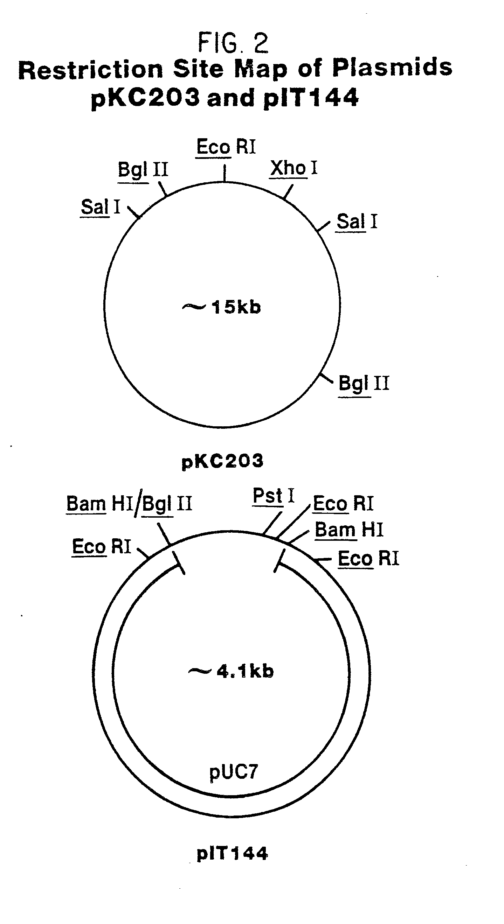

Plasmid pKC222, from which the DNA of the present invention can be obtained, is ~6.8 kb and is constructed by ligating the ~2.75 kg *Sal*I-*Bgl*II fragment of plasmid pKC203 to the ~4.1 kb *Sal*I-*Bgl*II fragment of plasmid pKC7. Plasmid pKC203 is ~15 kb and can be conventionally isolated from *E. coli* JR225, a strain deposited and made part of the permanent stock culture collection of the American Type Culture Collection, Rockville, Maryland. The strain is available to the public as a preferred source and stock reservoir of plasmid pKC203 under the accession number ATCC 31912. Plasmid pKC7 is known in the art (ATCC 37084) and can also be constructed in accordance with the procedure disclosed in Rao and Rogers, 1979, Gene 7:79. A restriction site map of each of plasmids pKC222 and pKC203 is presented respectively in Figures 1 and 2 of the accompanying drawings.

The DNA of the present invention is useful as a dominant selectable marker when fused in translational reading phase with a transcriptional and translational activator sequence-containing gene or portion of a gene. The number of amino acids encoded by the gene or portion of a gene is not critical for purposes of the present invention. In the case of a bacterial gene, such a fusion can be made by ligating the truncated aph(4) gene of plasmid pIT123 into plasmic pUC7. Plasmid pUC7, commercially available and constructed in substantial accordance with the teaching of Vieira and Messing, 1982, Gene 9:259, contains a portion of the *E. coli* lac Z gene and also a unique *Bam*HI restriction site downstream from the lac operator and translation initiation signals. The reading frame at the *Bam*HI site within the lac Z gene fragment is the same as that required for the truncated aph(4) gene of plasmid pIT123. Accordingly, joining the two genes

at the *Bam*HI site by ligating the ~1.3 kb *Bam*HI-*Bgl*II fragment of plasmid pIT123 into BamHI digested plasmid pUC7 results in a hybrid gene that is capable of conferring resistance to hygromycin B. Such illustrative construction comprises the coding sequence for the first twelve amino acids of lac Z fused with truncated aph(4)-gene. A restriction site map of the resultant gene-containing plasmid, designated as pIT144, is presented in Figure 2 of the accompanying drawings. A similar plasmid, designated as pKC307, was constructedby ligating blunt ended *Hph*I-digested plasmid pIT104 into blunt ended *Hinc*II-digested plasmid pUC8. The latter plasmid is similar to plasmid pUC7 and is also commercially available and constructed in substantial accordance with the teaching of Vieira and Messing, 1982.

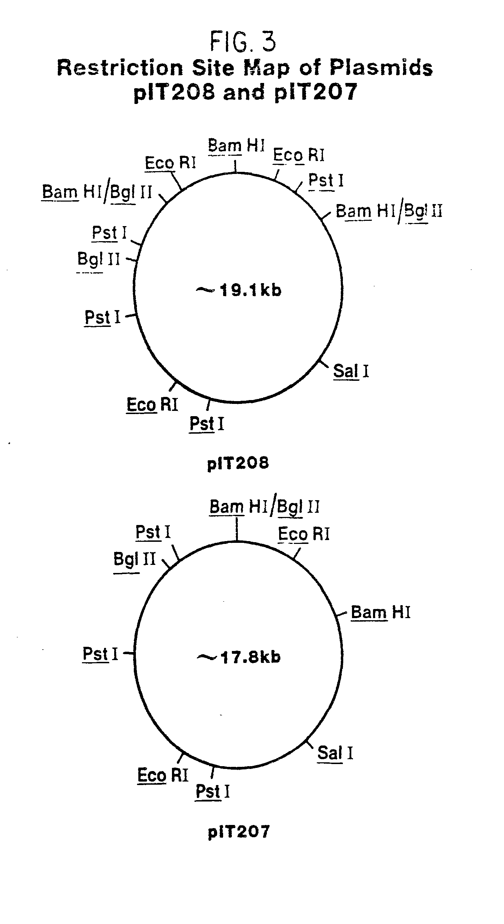

The DNA of the present invention can also be fused with eukaryotic genes or portions of genes, such as, for example, the yeast heat shock gene (YG101), disclosed in Ingolia *et al.,* 1982, Mol. and Cellular Biol. 2:1388. The ability to mobilize the transcriptional and translational activator sequences of YG101 on a 750 bp *Bam*HI-*Bgl*II fragment of plasmid pIT118 allows for an especially convenient fusion. This is done by first constructing plasmid pIT207, an intermediate plasmid comprising the aforementioned transcriptional and translational activator sequence-containing fragment ligated into *Bam*HI restricted plasmid pMC1587. Ligation of the ~1.3 kg *Bam*HI-*Bgl*II fragment of plasmid pIT123 into *Bam*HI-digested plasmid pIT207 results in the bifunctional plasmid pIT208. Plasmid pIT208 is selectable in *E. coli*, confers resistance to antibiotic hygromycin B in yeast and thus is illustrative of the present invention. A restriction site map of plasmid pIT208 is presented in Figure 3 of the accompanying drawings.

The heat shock gene (YG100), also disclosed in Ingolia *et al.,* 1982, can similarly be used for constructing convenient translational fusions. This is done by ligating the transcriptional and translational activator sequence-containing ~1 kb *Bam*HI-*Bgl*II fragment of plasmid pIT120 into *Bam*HI-digested plasmid pIT213. The latter plasmid comprises the known plasmid pRB5, a kanamycin resistance gene and the aforementioned truncated hygromycin B resistance gene-containing ~1.3 kb *Bam*HI-*Bgl*II fragment of plasmid pIT123. Plasmid pIT120, from which the hs100 transcriptional and translational activator sequence can be obtained, can be conventionally isolated from *E. coli* K12 JA221/pIT120, a strain deposited and made part of the permanent stock culture collection of the Northern Regional Research Laboratory, Peoria, Illinois. The strain is available to the public as a preferred source and stock reservoir of the plasmid under the accession number NRRL B—15603. The aforementioned ligation of the pIT120 and pIT213 fragments results in the illustrative bifunctional plasmid pIT217. Plasmid pIT217 is selectable in *E. coli*, confers resistance to hygromycin B in yeast and thus further exemplifies the present invention.

Those skilled in the art will recognize that ligation of the aforementioned *Bam*HI-digested plasmid pIT213 and the 750 bp *Bam*HI-*Bgl*II fragment of plasmid pIT118 results in an illustrative fusion which is also within the scope of the present invention. The resultant plasmid, designated as pIT215, is selectable in *E. coli* and confers hygromycin B resistance in yeast. Additional constructions employing different genes can also be made. For example, the eukaryotic phosphoglycerate kinase gene (PGK) can be fused with the present truncated hygromycin B resistance-conferring DNA by ligating the transcriptional and translational activator sequence-containing 230 bp *Bam*HI fragment of plasmid pIT143 into *Bam*HI-digested plasmid pIT213. Plasmid pIT143, from which the PGK transcriptional and translational activator sequence is obtained, is constructed by digesting the 958 bp *Cla*I-*Hinc*II fragment of plasmid pIT141 with the restriction enzyme *Mbo*II, removing the resultant extensions with the Klenow fragment of DNA polymerase, attaching *Bam*HI linkers with the sequence TGGATCCA and then ligating the linker-containing fragment into *Bam*HI-digested plasmid pUC8. Plasmid pIT141, which contains the entire PGK gene, is used to construct plasmid pIT143 and can be conventionally isolated from *E. coli* K12 JA221/pIT141, a strain deposited and made part of the permanent stock culture collection of the Northern Regional Research Laboratory, Peoria, Illinois. The strain is available to the public as a preferred source and stock reservoir of the plasmid under the accession number NRRL B—15602.

Those skilled in the art will recognize that a wide variety of genes or portions of genes can be substituted for the illustrative bacterial lac Z and eukaryotic PGK, YG100 and YG101 genes exemplified above. The number of amino acids encoded by such genes or portions of genes is not critical for purposes of the present invention. Other genes include genes from 1) *E. coli*, such as, for example, the trpE and lipoprotein genes; 2) *Saccharomyces cerevisiae*, such as for example, the alpha factor gene; 3) *Bacillus*, such as, for example, the alpha amylase and spoVG genes; 4) *Streptomyces* such as, for example, the thiostrepton resistance, neomycin resistance and viomycin resistance genes; 5) viruses or bacteriophages, such as, for example, the genes transcribed by $\lambda P_L$ and $\lambda P_R$ promoters; 6) mammals, such as, for example, the thymidine kinase and dihydrofolate reductase genes; and 7) plants, such as, for example, the octopine synthetase and nopaline synthetase genes.

The aforementioned genes can be truncated by treatment with an appropriate restriction enzyme and/or *Bal*31 nuclease and then, depending upon convenience and the particular fusion desired, provided with molecular linkers. Molecular linkers are commercially available or, if a special or unusual gene fusion is desired, constructed in accordance with the teaching of Itakura *et al.,* 1977 and Crea *et al.* 1978. A particularly useful fusion results from ligating the ~1.3 kb truncated aph(4) gene-containing fragment of plasmid pIT123 into the ~4.5 kb *Bam*HI-*Bgl*II fragment of plasmid pIA7Δ4Δ1. The latter fragment contains the transcriptional and translational activator sequence and also a 15 amino acid coding region of the bacterial trp LE' gene. The aforementioned ligation results in the illustrative ~5.8 kb plasmid pIT125. Those skilled in the art will recognize that fusing the other aforementioned truncated genes, with or without molecular linkers, to the present truncated aph(4) gene also results in vectors illustrative and within the scope of the present invention.

Vectors comprising the present DNA can be used in any hygromycin B sensitive host cell provided 1) that the vector replicates in the host cell or is integrated into the host cell chromosome; 2) that the gene fused to the truncated aph(4) gene is expressed in the host cell; and 3) that the host cell is susceptible to transformation. Illustrative and particularly useful host cells include, for example, *E. coli, E. coli* K12, *E. coli* K12 JA221, *E. coli* K12 HB101, *E. coli* K12 RR1, *Streptomyces, Streptomyces ambofaciens, Bacillus, Bacillus subtilis, Saccharomyces cerevisiae,* mammalian cells and plant cells, especially Angiospermous cells. Those skilled in the art will recognize that other host cells transformed by vectors comprising the present DNA are also illustrative and within the scope of the present invention.

While all the embodiments of the present invention are useful, some of the present DNA sequences, cloning vectors and transformants are preferred. Accordingly, preferred DNA sequences are the ~1.3 kb *Bam*HI-*Bgl*II fragment of plasmid pIT123 and the sequence

```
            CCT GAA CTC ACC GCG ACG TCT GTC GAG AAG
            ||| ||| ||| ||| ||| ||| ||| ||| ||| |||
            GGA CTT GAG TGG CGC TGC AGA CAG CTC TTC

TTT CTG ATC GAA AAG TTC GAC AGC GTC TCC GAC CTG ATG
||| ||| ||| ||| ||| ||| ||| ||| ||| ||| ||| ||| |||
AAA GAC TAG CTT TTC AAG CTG TCG CAG AGG CTG GAC TAC

CAG CTC TCG GAG GGC GAA GAA TCT CGT GCT TTC AGC TTC
||| ||| ||| ||| ||| ||| ||| ||| ||| ||| ||| ||| |||
GTC GAG AGC CTC CCG CTT CTT AGA GCA CGA AAG TCG AAG

GAT GTA GGA GGG CGT GGA TAT GTC CTG CGG GTA AAT AGC
||| ||| ||| ||| ||| ||| ||| ||| ||| ||| ||| ||| |||
CTA CAT CCT CCC GCA CCT ATA CAG GAC GCC CAT TTA TCG

TGC GCC GAT GGT TTC TAC AAA GAT CGT TAT GTT TAT CGG
||| ||| ||| ||| ||| ||| ||| ||| ||| ||| ||| ||| |||
ACG CGG CTA CCA AAG ATG TTT CTA GCA ATA CAA ATA GCC

CAC TTT GCA TCG GCC GCG CTC CCG ATT CCG GAA GTG CTT
||| ||| ||| ||| ||| ||| ||| ||| ||| ||| ||| ||| |||
GTG AAA CGT AGC CGG CGC GAG GGC TAA GGC CTT CAC GAA

GAC ATT GGG GAA TTC AGC GAG AGC CTG ACC TAT TGC ATC
||| ||| ||| ||| ||| ||| ||| ||| ||| ||| ||| ||| |||
CTG TAA CCC CTT AAG TCG CTC TCG GAC TGG ATA ACG TAG

TCC CGC CGT GCA CAG GGT GTC ACG TTG CAA GAC CTG CCT
||| ||| ||| ||| ||| ||| ||| ||| ||| ||| ||| ||| |||
AGG GCG GCA CGT GTC CCA CAG TGC AAC GTT CTG GAC GGA

GAA ACC GAA CTG CCC GCT GTT CTG CAG CCG GTC GCG GAG
||| ||| ||| ||| ||| ||| ||| ||| ||| ||| ||| ||| |||
CTT TGG CTT GAC GGG CGA CAA GAC GTC GGC CAG CGC CTC

GCC ATG GAT GCG ATC GCT GCG GCC GAT CTT AGC CAG ACG
||| ||| ||| ||| ||| ||| ||| ||| ||| ||| ||| ||| |||
CGG TAC CTA CGC TAG CGA CGC CGG CTA GAA TCG GTC TGC

AGC GGG TTC GGC CCA TTC GGA CCG CAA GGA ATC GGT CAA
||| ||| ||| ||| ||| ||| ||| ||| ||| ||| ||| ||| |||
TCG CCC AAG CCG GGT AAG CCT GGC GTT CCT TAG CCA GTT

TAC ACT ACA TGG CGT GAT TTC ATA TGC GCG ATT GCT GAT
||| ||| ||| ||| ||| ||| ||| ||| ||| ||| ||| ||| |||
ATG TGA TGT ACC GCA CTA AAG TAT ACG CGC TAA CGA CTA

CCC CAT GTG TAT CAC TGG CAA ACT GTG ATG GAC GAC ACC
||| ||| ||| ||| ||| ||| ||| ||| ||| ||| ||| ||| |||
GGG GTA CAC ATA GTG ACC GTT TGA CAC TAC CTG CTG TGG

GTC AGT GCG TCC GTC GCG CAG GCT CTC GAT GAG CTG ATG
||| ||| ||| ||| ||| ||| ||| ||| ||| ||| ||| ||| |||
CAG TCA CGC AGG CAG CGC GTC CGA GAG CTA CTC GAC TAC
```

```
CTT TGG GCC GAG GAC TGC CCC GAA GTC CGG CAC CTC GTG
!!! !!! !!! !!! !!! !!! !!! !!! !!! !!! !!! !!! !!!
GAA ACC CGG CTC CTG ACG GGG CTT CAG GCC GTG GAG CAC

CAC GCG GAT TTC GGC TCC AAC AAT GTC CTG ACG GAC AAC
!!! !!! !!! !!! !!! !!! !!! !!! !!! !!! !!! !!! TTG
GTG CGC CTA AAG CCG AGG TTG TTA CAG GAC TGC CTG

AAT GGC CGC ATA ACA GCG GTC ATT GAC TGG AGC GAG GCG
!!! !!! !!! !!! !!! !!! !!! !!! !!! !!! !!! !!! !!!
TTA CCG GCG TAT TGT CGC CAG TAA CTG ACC TCG CTC CGC

ATG TTC GGG GAT TCC CAA TAC GAG GTC GCC AAC ATC TTC
!!! !!! !!! !!! !!! !!! !!! !!! !!! !!! !!! !!! !!!
TAC AAG CCC CTA AGG GTT ATG CTC CAG CGG TTG TAG AAG

TTC TGG AGG CCG TGG TTG GCT TGT ATG GAG CAG CAG ACG
!!! !!! !!! !!! !!! !!! !!! !!! !!! !!! !!! !!! !!!
AAG ACC TCC GGC ACC AAC CGA ACA TAC CTC GTC GTC TGC

CGC TAC TTC GAG CGG AGG CAT CCG GAG CTT GCA GGA TCG
!!! !!! !!! !!! !!! !!! !!! !!! !!! !!! !!! !!! !!!
GCG ATG AAG CTC GCC TCC GTA GGC CTC GAA CGT CCT AGC

CCG CGG CTC CGG GCG TAT ATG CTC CGC ATT GGT CTT GAC
!!! !!! !!! !!! !!! !!! !!! !!! !!! !!! !!! !!! !!!
GGC GCC GAG GCC CGC ATA TAC GAG GCG TAA CCA GAA CTG

CAA CTC TAT CAG AGC TTG GTT GAC GGC AAT TTC GAT GAT
!!! !!! !!! !!! !!! !!! !!! !!! !!! !!! !!! !!! !!!
GTT GAG ATA GTC TCG AAC CAA CTG CCG TTA AAG CTA CTA

GCA GCT TGG GCG CAG GGT CGA TGC GAC GCA ATC GTC CGA
!!! !!! !!! !!! !!! !!! !!! !!! !!! !!! !!! !!! !!!
CGT CGA ACC CGC GTC CCA GCT ACG CTG CGT TAG CAG GCT

TCC GGA GCC GGG ACT GTC GGG CGT ACA CAA ATC GCC CGC
!!! !!! !!! !!! !!! !!! !!! !!! !!! !!! !!! !!! !!!
AGG CCT CGG CCC TGA CAG CCC GCA TGT GTT TAG CGG GCG

AGA AGC GCG GCC GTC TGG ACC GAT GGC TGT GTA GAA GTA
!!! !!! !!! !!! !!! !!! !!! !!! !!! !!! !!! !!! !!!
TCT TCG CGC CGG CAG ACC TGG CTA CCG ACA CAT CTT CAT

CTC GCC GAT AGT GGA AAC CGA CGC CCC AGC ACT CGT CCG
!!! !!! !!! !!! !!! !!! !!! !!! !!! !!! !!! !!! !!!
GAG CGG CTA TCA CCT TTG GCT GCG GGG TCG TGA GCA GGC

AGG GCA AAG GAA TAG
!!! !!! !!! !!! !!!
TCC CGT TTC CTT ATC
```

wherein

A is deoxyadenyl,
G is deoxyguanidyl,
C is deoxycytidyl and
T is thymidyl;

preferred plasmids are plasmids pIT123, pIT125, pKC307, pIT208, pIT215, pIT217, pIT219 and pIT144; and preferred transformants are E. coli K12 JA221/pIT123, E. coli K12 JA221/pIT125, E. coli K12 JA221/pKC307, E. coli K12 JA221/pIT208, E. coli K12 JA221/pIT215, E. coli K12 JA221/pIT217, E. coli K12 JA221/pIT219, E. coli K12 JA221/pIT144 and Saccharomyces cerevisiae/pIT208, Saccharomyces cerevisiae/pIT215, Saccharomyces cerevisiae/pIT217 and Saccharomyces cerevisiae/pIT219.

The DNA of the present invention is useful as a selectable marker in both homologous (E. coli) and heterologous (non-E. coli) systems and thus allows for the construction of selectable vehicles for cloning genes into host cells of diverse nature. The ability of the present DNA to confer resistance to antibiotic hygromycin B also provides a functional test for selecting transformants. This is important because of the practical necessity for determining and selecting the particular cells that have acquired the vector DNA. Additional DNA segments, that lack functional tests for their presence, can be inserted into the vectors and then transformants containing the non-selectable DNA can be isolated by antibiotic hygromycin B selection. Such non-selectable DNA segments include, but are not limited to, genes that specify human insulin A chain, human insulin B chain, human proinsulin, human preproinsulin, human growth hormone, bovine growth hormone, porcine growth hormone, avian growth hormone, human interferon and non-human interferon.

More particularly, a non-selectable DNA segment that comprises a gene is inserted on a plasmid, such as, for example, illustrative plasmid pIT144 or pIT208. The non-selectable DNA can be inserted into one of the *Eco*RI sites of pIT144 after a partial digestion of pIT144 with *Sal*I or into the *Sma*I site of pIT208. The desired recombinant is identified by colony hybridization of cells transformed with the ligation mixture using a nick translated probe. After confirming the presence of the non-selectable DNA, the vectors are further amplified in *E. coli* and then, in the case of plasmid pIT208, introduced into yeast. Yeast transformants are readily identified by antibiotic hygromycin B selection. Therefore, the ability to select for antibiotic resistance allows for the efficient isolation of the extremely rare cells that contain the particular non-selectable DNA of interest.

The functional test for hygromycin B resistance, as described herein above, is also used to identify DNA segments that can act as control elements for directing gene expression. Such segments, including but not limited to, promoters, attenuators, repressors, inducers, ribosomal binding sites, and the like, are used to control the expression of economically important genes. In addition, the present invention is useful for isolating and identifying origins of replication. This is done by cloning DNA fragments into vectors that contain the present aph(4) gene fusion and then transforming appropriate host cells under conditions of hygromycin B selection. Hygromycin B resistant cells can then be selected and the DNA used to transform *E. coli*, thus facilitating isolation of replicons from practically any organism of interest.

The resistance-conferring vectors of the present invention are also useful for insuring that linked DNA fragments are stably maintained in *E. coli*, yeast and other transformants. These genes or DNA fragments, covalently linked to the present aph(4) gene fusion, are maintained by exposing the transformants to levels of hygromycin B that are toxic to non-transformed cells. Therefore, transformants that lose the vector, and consequently any covalently linked DNA, cannot grow and are eliminated from the culture. This is particularly important in large scale fermentation where the maximum efficiency of product expression is desired.

The present DNA, cloning vectors and transformants are particularly useful for cloning genes which directly or indirectly encode specific functional polypeptides or fused gene products such as, for example, human insulin A chain, human insulin B chain, human proinsulin, human preproinsulin, human growth hormone, non-human growth hormone, human and non-human interferon, and the like; enzymatic functions in metabolic pathways leading to commercially important processes and compounds; control elements that improve gene expression or vector replication; or any physiologically active enzyme of research or commercial value. DNA sequences encoding enzymatic functions include, but are not limited to, sequences that code for enzymes that catalyze synthesis of cephalosporin antibiotics, actaplanin, penicillin, penicillin derivatives and tylosin. Those skilled in the art will understand that the present invention is broadly applicable and thus not limited to the cloning of the particular genes specified above.

The following examples further illustrate and detail the invention disclosed herein. Both an explanation of and the actual procedures for constructing the invention are described where appropriate.

Example 1
Construction of Plasmid pKC222 Starting Material
A. Isolation of Plasmid pKC203 and Construction of *E. coli* K12 BE827/pKC203

The bacterium *E. coli* JR225 (ATCC No. 31912) was cultured in TY broth (1% tryptone, .5% yeast extract, .5% sodium chloride, pH 7.4) with 100 µg./ml. of antibiotic hygromycin B according to conventional microbiological procedures. After 18 hours incubation, about .5 ml. of the culture was transferred to a 1.5 ml. Eppendorf tube and centrifuged for about 15 seconds. Unless otherwise indicated, all the manipulations were done at ambient temperature. The resultant supernatant was carefully removed with a fine-tip aspirator and the cell pellet was suspended in about 100 µl. of freshly prepared lysozyme solution which contained 2 mg./ml. lysozyme, 50 mM glucose, 10 mM CDTA (cyclohexane diaminetetracetate) and 25 mM Tris-HCl (pH 8.0). After incubation at 0°C. for 30 minutes, about 200 µl. of alkaline SDS (sodium dodecyl sulfate) solution (.2N NaOH, 1% SDS) were added and the tube was gently vortexed and then maintained at 0°C. for 15 minutes. Next, about 150 ml. of 3M sodium acetate (prepared by dissolving 3 moles of sodium acetate in a minimum of water, adjusting the pH to 4.8 with glacial acetic acid, and then adjusting the volume to 1 l.) were added and the contents of the tube were then mixed gently by inversion for a few seconds during which time a DNA clot formed.

The tube was maintained at 0°C. for 60 minutes and then centrifuged for 5 minutes to yield an almost clear supernatant. About .4 ml. of the supernatant was transferred to a second centrifuge tube to which 1 ml. of cold ethanol was added. After the tube was held at −20°C. for 30 minutes, the resultant precipitate was collected by centrifugation (2 minutes) and the supernatant was removed by aspiration. The thus collected pellet was dissolved in 100 µl. of .1M sodium acetate/.05M Tris-HCl (pH 8) and was reprecipitated by the addition of 2 volumes of cold ethanol. After 10 minutes at 20°C., the desired *E. coli* JR225 plasmid DNA precipitate was collected by centrifugation as described above.

The *E. coli* JR225 plasmid DNA pellet was dissolved in about 40 µl. of water or dilute buffer, and then used to transform *E. coli* K12 BE827 in substantial accordance with the transformation method of Wensink, 1974, Cell 3:315. *E. coli* K12 BE827 has been deposited and made part of the permanent stock culture collection of the American Type Culture Collection, Rockville, Maryland, from which it is available to the public under the number ATCC 31911. The resultant transformants were selected on TY agar (1% tryptone, .5% yeast extract, .5% sodium chloride, 1.5% agar, pH 7.4) containing 200 µg./ml. of antibiotic hygromycin B. Some of the transformants, as shown by gel electrophoresis (Rao and Rogers, 1978, Gene 3:247) and other tests, contained both large and smaller (~15 kb) plasmids and were resistant to both antibiotics ampicillin and hygromycin B. Other transformants contained only the smaller ~15 kb plasmid and were resistant to antibiotics hygromycin B and G418 but were sensitive to ampicillin.

Transformants of the latter type were plated on TY agar containing 0.1 mg./ml. of antibiotic hygromycin B and were cultured using standard microbiological techniques. The resultant cells were used to isolate the above described ~15 kb hygromycin B and G418 resistance-conferring plasmid, hereinafter designated as plasmid pKC203. The presence of the antibiotic hygromycin B and G418 resistance genes on plasmid pKC203 was confirmed by subsequent transformation and selection analysis.

B. Construction of Plasmid pKC222 and Transformant *E. coli* K12 JA221/pKC222

1. Isolation of the ~2.75 kb *Sal*I/*Bgl*II Fragment Plasmid pKC203

About 5 µg. of plasmid pKC203 DNA were treated with *Sal*I and *Bgl*II restriction enzymes according to the instructions and under the conditions specified by the manufacturer\*. In addition, useful procedures for restriction enzyme digestion are also disclosed in Maniatis *et al.*, 1982, Molecular Cloning, Cold Spring Harbor Laboratory, Cold Spring Harbor, New York. An ~3.76 kb fragment that contained the genes and control elements for resistance to antibiotics hygromycin B and G418 were recovered by conventional procedures (Maniatis *et al.,* 1982).

2. Ligation and Final Construction

About 5 µg. of plasmid pKC7 (ATCC 37084) which can be constructed in accordance with the teaching of Rao and Rogers, 1979, Gene 7:79, were treated with *Sal*I and *Bgl*II restriction enzymes. After the enzymes were inactivated by heating at 70°C. for 5 minutes, about 1 µg. of the DNA was mixed in a 1:1 ratio with the ~2.75 kb *Sal*I/*Bgl*II fragment of pKC203. The fragments were joined using T4 DNA ligase according to the instructions and under the conditions specified by the manufacturer as cited in Example 1B-1. In addition, useful procedures for both restriction enzyme digestion and ligation are also disclosed in Maniatis *et al.,* 1982. The resulting plasmid pKC222 was transformed into *E. coli* K12 JA221 (NRRL B—15211) in substantial accordance with the teaching of Example 1A. The resultant transformants were selected on TY agar (1% typtone, .5% yeast extract, .5% NaCl, 1.5% agar) containing 50 µg./ml. of antibiotic ampicillin. Transformants were then screened for the desired plasmid.

3. Isolation of Plasmid pKC222

Purified transformants were cultured in TY broth (1% tryptone, .5% yeast extract, .5% sodium chloride, pH 7.4) with 50 µg./ml. of antibiotic ampicillin according to conventional microbiological procedures. After 18 hours incubation, about .5 ml. of the culture was transferred to a 1.5 ml. Eppendorf tube and centrifuged for about 15 seconds. Unless otherwise indicated, all the manipulations were done at ambient temperature. The resultant supernatant was carefully removed with a fine-tip aspirator and the cell pellet was suspended in about 100 µl. of freshly prepared lysozyme solution which contained 2 mg./ml. lysozyme, 50 mM glucose, 10 mM CDTA (cyclohexane diaminetetraacetate) and 25 mM Tris-HCl (pH 8). After incubation at 0°C. for 30 minutes, about 200 µl. of alkaline SDS (sodium dodecyl sulfate) solution (.2N NaOH, 1% SDS) were added and the tube was gently vortexed and then maintained at 0°C. for 15 minutes. Next, about 150 µl. of 3M sodium acetate (prepared by dissolving 3 moles of sodium acetate in a minimum of water, adjusting the pH to 4.8 with glacial acetic acid and then adjusting the volume to 1 l.) were added and the contents mixed gently for a few seconds by inversion. A DNA clot formed, after which the resultant mixture was maintained at 0°C. for 60 minutes and then centrifuged for 5 minutes to yield an almost clear supernatant. About .4 ml. of the supernatant were transferred to a second centrifuge tube to which 1 ml. of cold ethanol was added. After the tube was held at −20°C. for 30 minutes, the resultant precipitate was collected by centrifugation (2 minutes) and the supernatant was removed by aspiration. The thus collected pellet was dissolved in 100 µl. of .1M sodium acetate/.05M Tris-HCl (pH 8) and was reprecipitated by the addition of 2 volumes of cold ethanol. After 10 minutes at −20°C., the precipitate was collected, as described above, by centrifugation and constituted the desired pKC222 DNA as determined by agarose gel electrophoresis (Rao and Rogers, 1978).

---

\*Unless otherwise indicated, restriction enzymes, T4 DNA ligase, DNA polymerase and Klenow fragment (including instructions for their use) can be obtained from the following source:

New England Biolabs.
32 Tozer Road
Beverly MA 01915

## Example 2
### Construction of Plasmid pIT123 and *E. coli* K12 JA221/pIT123
#### A. Isolation of the *Hph*I-*Pst*I Fragment of Plasmid pKC222

About 50 µg. of plasmid pKC222 DNA were digested in 1× *Hph*I salts (6 mM KCI, 10 mM Tris-HCI, pH 7.4, 10 mM $MgCl_2$, 1 mM dithiothreitol) in a total volume of 100 µl. with 20 New England Biolab Units of *Hph*I restriction endonuclease. Completion of digestion was checked by electrophoresing 2% of the reaction mixture on agarose. After the NaCl concentration was adjusted to 60 mM by addition of an appropriate volume of 5M NaCl, about 20 units of *Pst*I restriction endonuclease were added. Completion of digestion was again monitored by agarose gel electrophoresis. The desired 325 bp (plus single stranded extensions) *Hph*I-*Pst*I fragment was purified from acrylamide using standard techniques (Schlief and Wensink, 1981, Practical Methods in Molecular Biology. Springer-Verlag, NY).

The purified 325 bp fragment was treated with *E. coli* DNA polymerase I large fragment (New England Biolabs). Thus, about 1.5 µl. (1 µg.) of fragment, .5 µl. of 10× buffer (.5M Tris, pH 7.5, .1M $MgCl_2$), .5 µl. each of (200 mM) dCTP, dATP, TTP and dGTP and 1 µl. (containing 1 unit) of DNA polymerase I large (Klenow) fragment were incubated at 37°C. for 15 minutes. After heat inactivation of the polymerase, *Bam*HI linkers were added in substantial accordance with the procedure of Roberts and Lauer, 1979, Methods in Enzymology 68:473. The resultant *Bam*HI linker-containing DNA was conventionally digested with *Bam*HI restriction enzyme in 1× *Bam*HI salts (.15M NaCl, 6 mM Tris-HCI, pH 7.9, 6 mM $MgCl_2$). Next, the Tris-HCI concentration was increased to 100 mM with an appropriate volume of 2M Tris-HCI, pH 7.4 and then the DNA was further digested with *Eco*RI restriction enzyme. The resultant digested DNA was again electrophoresed on a 7% acrylamide gel and the desired 250 bp fragment was purified as before.

#### B. Construction of Plasmid pIT122 and *E. coli* K12 JA221/pIT122

About 2 µg. of pBR322 DNA were sequentially digested with *Bam*HI and *Eco*RI restriction enzymes in substantial accordance with the teaching of Example 2A. After the enzymes were inactivated by heating at 70°C. for 5 minutes, about 1 µl. (1 µg.) of the pBR322 DNA was mixed with about 1 µl. (1 µg.) of the purified 250 bp fragment, 37 µl. water, 5 µl. (10 mM) ATP, 5 µl. ligation mix (.5M Tris-HCI, pH 7.8, .1M dithiothreitol, .1M $MgCl_2$), and 1 µl. T4 DNA ligase (approximately 100,000 New England Biolabs Units). The mixture was incubated at 15°C. for about 2 hours and then the reaction was terminated by incubation at 70°C. for 5 minutes. After cooling on ice, the resultant ligated mixture was used to transform, in substantial accordance with the transformation procedure of Wensink, 1974, *E. coli* K12 JA221 (NRRL B—15211) on TY plates containing ampicillin at 200 µg./ml. The identity of the desired transformants was conventionally confirmed by testing for the expected phenotype ($Amp^R$, $Tet^S$) and also for the appropriate *Eco*RI-*Bam*HI insert. The resultant *E. coli* K12 JA221/pIT122 transformants were conventionally cultured for subsequent production and isolation of plasmid pIT122.

#### C. Ligation of ~1.45 kb *Eco*RI Fragment of Plasmid pKC222 into *Eco*RI-Digested Plasmid pIT122

About 20 µg. of plasmids pKC222 and pIT122 were independently cleaved in separate reaction volumes of 200 µl. each with 40 units of *Eco*RI restriction enzyme in 1× *Eco*RI reaction mix (.1M Tris-HCI, pH 7.5, .05M NaCl, 0.005M $MgCl_2$). The desired ~1.45 kb *Eco*RI fragment was conventionally purified from a 7% acrylamide gel and ligated into the *Eco*RI-digested pIT122. The resultant ligated DNA was designated as plasmid pIT123 and was then used to transform *E. coli* K12 JA221 (NRRL B—15211). Both the ligation and transformation procedures were carried out in substantial accordance with the teaching of Example 2B. The ampicillin resistant transformants were conventionally screened for the presence and correct orientation of the ~1.45 kb *Eco*RI fragment by restriction enzyme and agarose gel electrophoretic analysis of the constitutive plasmids. Plasmids containing the entire pah(4) gene, except for first 9 base pairs, constituted the desired plasmid pIT123. The thus identified *E. coli* K12 JA221/pIT123 transformants were then cultured for subsequent production and isolation of plasmid pIT123. A restriction site map of plasmid pIT123 is presented in Figure 1 of the accompanying drawings.

## Example 3
### Construction of Plasmid pIT144 and *E. coli* K12 RR1ΔM15/pIT144
#### A. Construction and Isolation of the ~1.33 kb *Bam*HI-*Bgl*II Fragment of Plasmid pIT123

The desired digestion and isolation were carried out in substantial accordance with the teaching of Example 2A except that *Bam*HI and *Bgl*II restriction enzymes and salts, rather than *Hph*I and *Pst*I restriction enzymes and salts, were used.

#### B. *Bam*HI Digestion of Plasmid pUC7

The desired digestion was done in substantial accordance with the teaching of Example 2A except that 1 µg. of plasmid pUC7 (commercially available from Bethesda Research Laboratories, 8717 Grovemont Circle, P.O. Box 6009, Gaithersburg, MD 20877), rather than the *Bam*HI linker-containing DNA, was used.

#### C. Ligation and Transformation

About 1 µg. of the ~1.3 kb *Bam*HI-*Bgl*II fragment of plasmid pIT123 was ligated into about 1 µg. of *Bam*HI-digested pUC7 and then the resultant mixture was used to transform *E. coli* K12 RR1ΔM15 (deposited and made part of the permanent stock culture collection of the National Regional Research Laboratory, Peoria, Illinois from which it can be obtained under the accession number NRRL B—15440). Both procedures are carried out in substantial accordance with the ligation and transformation teachings of Example 2B. The transformed cells were plated onto TY plates containing 50 µg./ml. ampicillin, 100 mM isopropylthio-β-D-galactoside (IPTG), and .02% 5-bromo-4-chloro-3-indolyl-β-D-galactoside (X-gal). White ampicillin-resistant colonies were plated on TY containing ampicillin (50 µg./ml.), hygromycin B (200 µg./ml.) and IPTG (100 mM). The hygromycin B resistant cells constituted the desired *E. coli* K12 RR1ΔM15/pIT144 transformants, the identity of which was further confirmed by restriction enzyme, agarose gel electrophoresis analysis of the constitutive plasmids. The resultant *E. coli* K12 RR1ΔM15/pIT144 transformants were then conventionally cultured for subsequent production and isolation of plasmid pIT144. Plasmid pIT144 can transform conventional *E. coli* strains such as, for example, *E. coli* K12, *E. coli* K12 RR1, *E. coli* K12 JA221 and *E. coli* K12 HB101 in substantial accordance with the transformation teaching of Example 2B. A restriction site map of plasmid pIT144 is presented in Figure 2 of the accompanying drawings.

Example 4
Construction of Plasmid pIT208 and *E. coli* K12 JA221/pIT208

A. Construction of Plasmid pIT207
1. Construction and Isolation of the ~750 bp *Bam*HI-*Bgl*II Fragment of Plasmid pIT118
a. Isolation of Plasmid pIT118
Plasmid pIT118 can be isolated from *E. coli* K12 JA221/pIT118 in substantial accordance with the teaching of Example 1A. The aforementioned strain has been deposited and made part of the permanent stock culture collection of the Northern Regional Research Laboratory, Peoria, Illinois, under the accession number NRRL B—15441.

b. Digestion
The desired digestion and isolation were carried out in substantial accordance with the teaching of Example 2A except that *Bam*HI and *Bgl*II restriction enzymes, rather than *Hph*I and *Pst*I restriction enzymes, were used.

2. *Bam*HI Digestion of Plasmid pMC1587
The desired digestion was carried out in substantial accordance with the teaching of Example 2A except that 2 µg. of plasmid pMC1587, rather than the *Bam*HI linker-containing DNA, were used. Plasmid pMC1587 can be conventionally isolated, in substantial accordance with the teaching of Example 1A, from *E. coli* K12 JA221/pMC1587, a strain deposited and made part of the permanent stock culture collection of the Northern Regional Research Laboratory, Peoria, Illinois, under the accession number NRRL B—15442. Since plasmid pMC1587 has a single *Bam*HI site, digestion is easily monitored by agarose gel electrophoresis. The appearance of a single band of about 16 kb signals complete digestion.

3. Ligation and Transformation of *E. coli* K12 JA221
About 1 µg. of the ~750 bp *Bam*HI-*Bgl*II fragment of plasmid pIT118 was ligated into about 1 µg. of *Bam*HI-digested plasmid pMC1587 and then the resultant ligation mixture was used to transform *E. coli* K12 JA221 (NRRL B—15211). Both procedures were carried out in substantial accordance with the ligation and transformation teachings of Example 2B. The ampicillin resistant transformants were conventionally screened for the presence and correct orientation of the ~750 bp fragment by restriction enzyme and agarose gel electrophoretic analysis of the constitutive plasmids. Plasmids with the *Bam*HI/*Bgl*II junction (formed by the ligation of the ~750 bp fragment), oriented closest to the leu 2 gene constituted the desired plasmid pIT207. The *E. coli* K12 JA221/pIT207 transformants were cultured for subsequent production and isolation of plasmid pIT207.

B. Final Construction of Plasmid pIT208
1. *Bam*HI Digestion of Plasmid pIT207
The desired digestion was carried out in substantial accordance with the teaching of Example 2A except that 1 µg. of plasmid pIT207, rather than the *Bam*HI linker-containing DNA, was used.

2. Ligation and Construction of *E. coli* K12 JA221/pIT208
About 2 µg. of the ~1.3 kb *Bam*HI-*Bgl*II fragment of plasmid pIT123 (prepared in Example 3A) were ligated into about 2 µg. of *Bam*HI-digested plasmid pIT207 and then the resultant ligation mixture was used to transform *E. coli* K12 JA221 (NRRL B—15211). Both procedures were carried out in substantial accordance with the ligation and transformation teachings of Example 2B. The ampicillin resistant transformants were conventionally screened for the presence and correct orientation of the ~1.3 kb fragment by restriction enzyme and agarose gel electrophoretic analysis of the constitutive plasmids. Plasmids with the internal *Eco*RI site of the ~1.3 kb fragment oriented closest to the leu 2 gene constituted the desired pIT208 plasmids. The *E. coli* K12 JA221/pIT208 transformants were cultured for subsequent production and isolation of plasmid pIT208.

Plasmid pIT208 contains 1) the truncated YG101 gene fused in translational reading to the DNA of the present invention; 2) the yeast leu 2 gene, allowing for selection of the plasmid via complementation of leu 2 auxotrophs; 3) the yeast 2 micron sequences, facilitating autonomous replication in yeast; 4) the origin of replication from plasmid pBR322, facilitating autonomous replication in *E. coli*; and 5) the β-lactamase gene from pBR322, facilitating plasmid selection in *E. coli*. A restriction site map of plasmid pIT208 is presented in Figure 3 of the accompanying drawings.

### Example 5
### Construction of *Saccharomyces cerevisiae*/pIT208

*Saccharomyces cerevisiae* cells were transformed with plasmid pIT208 in substantial accordance with the teaching of Hinnen *et al.,* 1978, Proc. Nat. Acad. Sci. USA 75:1929. Although any yeast can be used, the particular strain exemplified herein is *Saccharomyces cerevisiae* DBY746. The strain is available to the public from the Yeast Genetics Stock Center, Department of Biophysics and Medical Physics, University of California, Berkeley, California 94720.

The desired construction was made by growing about 100 ml. of yeast cells at 30°C. in YPD medium (1% Bacto-yeast extract, 2% Bacto-peptone, and 2% D-glucose) to an A600 of about 1. Under sterile conditions, the cells were centrifuged and washed twice in 15 ml. of 1.2M sorbitol and then resuspended in 15 ml. of the sorbitol solution. After about 100 μl. of 2.5 mg./ml. zymolyase* (60,000 units in 5 mM KP04, pH 7.6, 1.2M sorbitol) were added, the cells were incubated at 30°C. The extent of protoplasting was monitored by adding 180 μl. of 10% SDS to 20 μl. aliquots of the incubated cells and then observing under phase contrast microscopy. When about 90% of the cells appeared black, the cells were harvested by gentle centrifugation, washed twice with 15 ml. of 1.2M sorbitol, resuspended in 10 ml. of 1.2M sorbitol-.5× YPD solution and incubated at room temperature for 40 minutes. The cells were again collected by gentle centrifugation and resuspended in 600 μl. of a solution comprising .5× YPD, 1.2M sorbitol, 10mM CaCl and 10mM Tris-HCl, pH 7.5. Aliquots (.2 ml) of these cells were removed and added to 20 μl. of the solution containing the DNA in 1.2M sorbitol. The mixture was incubated at room temperature for 10 minutes, at which time 1 ml. of a solution comprising 20% PEG 4000**, 10 mM $CaCl_2$, 10 mM Tris-HCl, pH 7.5 was added. The mixture was incubated at room temperature for 60 minutes and then divided into four portions. Each portion was added to tubes containing 25 ml. of 3% agar, .67% Difco yeast nitrogen base without amino acids, 1.2M sorbitol, 2% glucose, 2% YPD and other conventional nutrients. In addition, histidine, uracil and tryptophan were also added to select for leucine prototrophy. The cells were gently mixed, immediately added to an empty sterile petri dish and, after the agar solidified, incubated at 30°C. under moist conditions. After about 3 days, leucine prototrophs were picked and streaked on YPD plates containing 500 μg./ml. hygromycin B. The resultant hygromycin B-resistant yeast cells constituted the desired *Saccharomyces cerevisiae*/pIT208 transformants. The identity of the transformants was further confirmed by restriction enzyme and agarose gel electrophoretic analysis of the constitutive plasmids.

### Example 6
### Construction of Plasmid pKC307 and *E. coli* K12 RR1ΔM15/pKC307

A. Construction of Plasmid pIT104 and *E. coli* K12 RR1/pIT104

About 1.5 μl (1 μg) of *Sac*I-cut plasmid pKC222, .5 μl of 10× buffer (.5M Tris, ph 7.5, .1M $MgCl_2$), .5 μl each of (200 mM) dCTP, dATP, TTP and dGTP and 1 μl (containing 1 unit of DNA polymerase I large (Klenow) fragment were incubated at 37°C for 15 minutes. After heat inactivation of the polymerase, *Bam*HI linkers*** were added in substantial accordance with the procedure of Roberts and Lauer, 1979. The resultant *Bam*HI linker-containing DNA was conventionally digested with *Bam*HI restriction enzyme and then ligated in substantial accordance with the procedure of Example 2B. After digestion with *Sac*I restriction enzyme to reduce the number of parental plasmids, the resultant plasmid pIT104 DNA was used to transform, in substantial accordance with the procedure of Wensink, 1974, *E. coli* K12 RR1 (NRRL B—15210). The transformed cells were plated on LB plates (Rosenberg and Court, 1979, Ann. Rev. Genet. 13:319)

---

*Zymolyase can be obtained from the following source:
> Miles Laboratory
> P.O. Box 2000
> Elkhart, IN 46515

**PEG 4000 can be obtained from the following source:
> Baker Biochemicals
> 222 Red School Lane
> Phillipsburg, NJ 08865

***BamHI linkers [d(CCGGATCCGG)1 can be obtained from the following source:
> Collaborative Research,
> 128 Spring Street,
> Lexington,
> Massachusetts 02173.

containing ampicillin at 50 µg/ml. The resultant ampicillin resistant *E. coli* K12 RR1/pIT104 cells were conventionally isolated and cultured for the subsequent production and isolation of plasmid pIT104.

The structure of plasmid pIT104 was confirmed by transformation, selection, restriction enzyme and sequence analysis.

### B. *Hph*I Digestion of Plasmid pIT104

The desired digestion was carried out in substantial accordance with the teaching of Example 2A except that plasmid pIT104, rather than plasmid pKC222, was used and, in addition, there was no subsequent *Pst*I digestion. The resultant plasmid pIT104 *Hph*I digest was used without purification.

### C. *Hinc*II Digestion of Plasmid pUC8

The desired digestion was carried out in substantial accordance with the teachings of Example 2A except that plasmid pUC8 (commercially available from Bethesda Research Laboratories, 8717 Grovemont Circle, P.O. Box 6009, Gaithersburg, MD 20877), and *Hinc*II restriction enzyme and reaction mix*, rather than plasmid pKC222 and *Hph*I and *Pst*I restriction enzymes and salts, were used. The resultant plasmid pUC8 *Hinc*II digest was used without purification.

### D. Removal of 3' Extensions with T4 DNA Polymerase

The 3' extension left by the *Hph*I digestion of Example 6B was removed for subsequent blunt end ligation. Thus, about 1 µg, of the plasmid pIT104 *Hph*I digest was incubated in 10 µl of a solution comprising 33 mM Tris-HCl, pH 7.8, 67 mM potassium acetate, 10 mM magnesium acetate, .5 mM dithiothreitol, .1 mg/ml BSA, 150 µM each of dCTP, dATP, dGTP and TTP and 3 units of T4 DNA polymerase at 37°C for about 5 minutes. The reaction was conventionally terminated by incubation at 65°C after addition of about 1 µl of 50 mM EDTA.

### E. Ligation and Construction of *E. coli* K12 RR1ΔM15/pKC307

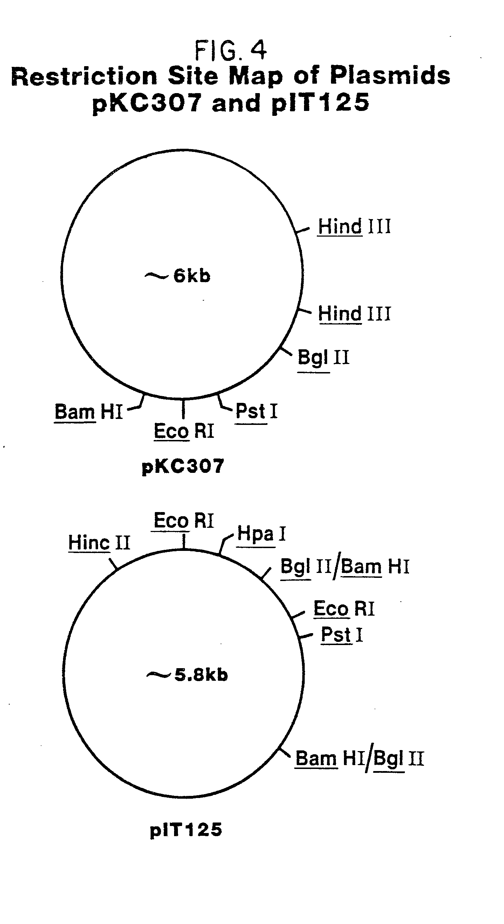

Ligation of the blunt ended plasmid pIT104 and plasmid pUC8 digests was conventionally carried out in substantial accordance with the ligation procedure of Maniatis *et al*. 1982. The resultant plasmid, designated as pKC307, was used to transform *E. coli* K12 RR1ΔM15 in substantial accordance with the transformation procedure of Example 2B and the selection procedure of Example 3C. The resultant *E. coli* K12 RR1ΔM15/pKC307 transformants were then conventionally cultured for subsequent production and isolation of plasmid pKC307. Plasmid pKC307 can transform conventional *E. coli* strains such as, for example, *E. coli* K12, *E. coli* K12 RR1, *E. coli* K12 JA221 and *E. coli* K12 HB101 in substantial accordance with the transformation teaching of Example 2B. A restriction site map of plasmid pKC307 is presented in Figure 4 of the accompanying drawings.

A functionally equivalent derivative of plasmid pKC307 was also prepared by conventionally deleting the ~1.7 kb *Hind*III fragment of the aforementioned plasmid. The resultant plasmid, designated as pKC308, was used to transform *E. coli* K12 JA221 and serves to further exemplify the present invention.

### Example 7
### Construction of Plasmid pIT125 and *E. coli* K12 JA221/pIT125

1. Construction of Plasmid pIA7Δ4Δ1

A. Construction of Plasmid pBRHtrp

Plasmid pGM1 carries the *E. coli* tryptophan operon containing the deletion ΔLE1413 (Miozzari, *et al*., 1978, *J. Bacteriology*, 1457—1466) and hence expresses a fusion protein comprising the first 6 amino acids of the trp leader and approximately the last third of the trp E polypeptide (hereinafter referred to in conjunction as LE'), as well as the trp D polypeptide in its entirety, all under the control of the trp promoteroperator system. *E. coli* K12 W3110*tna*2trpΔ102/pGM1 has been deposited with the American Type Culture Collection (ATCC No. 31622) and pGM1 may be conventionally removed from the strain for use in the procedures described below.

About 20 µg of the plasmid were digested with the restriction enzyme *Pvu*II which cleaves the plasmid at five sites. The gene fragments were next combined with *Eco*RI linkers (consisting of a self complementary oligonucleotide of the sequence: pCATGAATTCATG) providing an *Eco*RI cleavage site for later cloning into a plasmid containing an *Eco*RI site. The 20 µg of DNA fragments obtained from pGM1 were treated with 10 units T$_4$ DNA ligase in the presence of 200 pico moles of the 5'-phosphorylated synthetic oligonucleotide pCATGAATTCATG and in 20 µl T$_4$ DNA ligase buffer (20 mM tris, pH 7.6, .5 mM ATP, 10 mM MgCl$_2$, 5 mM dithiothreitol) at 4°C overnight. The solution was then heated 10 minutes at 70°C to halt ligation. The linkers were cleaved by *Eco*RI digestion and the fragments, now with *Eco*RI ends, were separated using 5 percent polyacrylamide gel electrophoresis (hereinafter "PAGE"). The three largest

---

*Reaction mix for *Hinc*II restriction enzyme was prepared with the following preferred composition:
    50 mM NaCl
    10 mM Tris-HCl, pH 7.5
    10 mM MgCl$_2$
    1 mM Dithiothreitol

fragments were isolated from the gel by first staining with ethidium bromide and then locating the fragments with ultraviolet light and cutting from the gel the portions of interest. Each gel fragment, with 300 microliters .1xTBE, was placed in a dialysis bag and subjected to electrophoresis at 100 v for one hour in .1xTBE buffer (TBE buffer contains: 10.8 g Tris base, 5.5 g boric acid, .09 g $Na_2EDTA$ in 1 liter $H_2O$). The aqueous solution was collected from the dialysis bag, phenol extracted, chloroform extracted, and made .2M with respect to sodium chloride. The DNA was then recovered in water after ethanol precipitation. The trp promoter/operator-containing gene with EcoRI sticky ends was identified in the procedure next described, which entails the insertion of fragments into a tetracycline sensitive plasmid which, upon promoter/operator insertion, becomes tetracycline resistant. All DNA fragment isolations hereinafter described are performed using PAGE followed by the electroelution method described above.

B. Construction of Plasmid pBRH trp Expressing Tetracycline Resistance Under the Control of the Trp Promoter/Operator and Identification and Amplification of the Trp Promoter/Operator Containing DNA Fragment Isolated in 'A' above

Plasmid pBRH1, (constructed in accordance with Rodriguez, et al., 1979, Nucleic Acids Research 6, 3267—3287 and West et al., 1979, Gene 7:271—288 and also deposited in the American Type Culture Collection under the accession number ATCC 37070) expresses ampicillin resistance and contains the gene for tetracycline resistance but, there being no associated promoter, does not express that resistance. The plasmid is accordingly tetracycline sensitive. By introducing a promoter/operator system in the EcoRI site, the plasmid can be made tetracycline resistant.

Plasmid pBRH1 (ATCC 37070) was digested with EcoRI. The enzyme was removed by phenol extraction followed by chloroform extraction and then the DNA was recovered in water after ethanol precipitation. The resulting DNA molecule was, in separate reaction mixtures, combined with each of the three DNA fragments obtained in Example 7A and ligated with $T_4$ DNA ligase as previously described. The DNA in the reaction mixture was used to transform competent E. coli K12 strain 294, (Backman et al., 1976, Proc. Nat. Acad. Sci. USA 73:4174—4198, ATCC No. 31446) by standard techniques (Hershfied et al., 1974, Proc. Nat. Acad. Sci. USA 71:3455—3459) and the bacteria were then plated on LB plates containing 20 µg/ml ampicillin and 5 µg/ml tetracycline.

Several tetracycline-resistant colonies were selected and the plasmid DNA was isolated and designated pBRHtrp. The presence of the desired fragment was confirmed by restriction enzyme analysis. Plasmid pBRH trp expresses β-lactamase, imparting ampicillin resistance, and contains a DNA fragment which includes the trp promoter/operator. The DNA fragment also codes for a first protein, (designated LE'), comprising a fusion of the first six amino acids of the trp leader and approximately the last third of the trp E polypeptide, a second protein (designated D'), corresponding to approximately the first half of the trp D polypeptide, and a third protein, coded for by the tetracycline resistance gene.

C. Construction of Plasmid pSOM7Δ2

Plasmid pBRHtrp was digested with EcoRI restriction enzyme and the resulting fragment, isolated by PAGE and electroelution, was combined with EcoRI-digested plasmid pSOM11 (Itakura et al., 1977, Sci. 198:1056, G.B. Patent Publication No. 2,007,676A). The mixture was ligated with $T_4$ DNA ligase and the resulting DNA transformed into E. coli K12 strain 294 as previously described. Transformant bacteria were selected on ampicillin-containing plates and the resulting ampicillin-resistant colonies were screened by colony hybridization (Gruenstein et al., 1975, Proc. Nat. Acad. Sci. USA 72:3951 3965). The trp promoter/operator-containing fragment, isolated from pBRH trp and then radioactively labelled with [32]p, was used as a probe in the above procedure. Several colonies were shown to be positive by colony hybridization and were therefore selected. Plasmid DNA was isolated and the orientation of the inserted fragments was determined by restriction analysis using enzymes BglII and BamHI in double digestion. Colonies containing the desired plasmid with the trp promoter/operator fragment in the proper orientation were grown in LB medium containing 10 µg/ml ampicillin. The desired plasmid was designated pSOM7Δ2 and was used for subsequent constructions described below.

D. Construction of Plasmid pTrp24

1. Construction of a Gene Fragment Comprising Codons for the Distal Regions of the LE' Polypeptide With BglII and EcoRI Restriction Sites Respectively at the 5' and 3' Ends of the Coding Strand

Plasmid pSOM7Δ2 was HindIII digested followed by digestion with lambda exonuclease (a 5' to 3' exonuclease) under conditions chosen so as to digest beyond the BglII restriction site within the LE' encoding region. About 20 µg of HindIII-digested pSOM7Δ2 was dissolved in buffer (20 mM glycine buffer, pH 9.6, 1 mM $MgCl_2$, 1 mM β-mercaptoethanol). The resulting mixture was treated with 5 units of lambda exonuclease for 60 minutes at room temperature. The reaction mixture obtained was then phenol extracted, chloroform extracted, and ethanol precipitated.

To create an EcoRI residue at the distal end of the LE' gene fragment, a primer [32]pCCTGTGCATGAT was synthesized by the improved phosphotriester method (Crea et al., 1978), and hybridized to the single stranded end of the LE' gene fragment resulting from lambda exonuclease digestion. The hybridization was performed by dissolving 20 µg of the lambda exonuclease-treated HindIII digestion product of plasmid pSOM7Δ2 in 20 µl. $H_2O$ and combining with 6 µl of a solution containing approximately 80 picomoles of the 5'-phosphorylated oligonucleotide described above. The synthetic fragment was hybridized to the 3' end of the LE' coding sequence and the remaining single strand portion of the LE' fragment was filled in by Klenow Polymerase I using dATP, TTP, dGTP and dCTP. Klenow Polymerase I is the fragment obtained by proteolytic cleavage of DNA Polymerase I. It contains the 5' → 3' polymerizing activity, the 3' → 5' exonucleolytic activity, but not the 5' → 3' exonucleolytic activity of the parental enzyme (Kornberg, 1974, W. E. Freeman and Co., San Francisco, California).

The reaction mixture was thus heated to 50°C and let cool slowly to 10°C, whereafter 4 µl of Klenow enzyme were added. After 15 minutes incubation at room temperature followed by 30 minutes incubation at 37°C, the reaction was stopped by the addition of 5 µl of .25M EDTA. The reaction mixture was phenol extracted, chloroform extracted, and ethanol precipitated. The DNA was subsequently cleaved with the restriction enzyme Bg/II and the fragments were separated by PAGE. An autoradiogram obtained from the gel revealed a $^{32}$P-labelled fragment, which was recovered by electroelution, of the expected length of approximately 470 bp. As outlined, this fragment LE'(d) has a Bg/II terminus and a blunt end coinciding with the beginning of the primer.

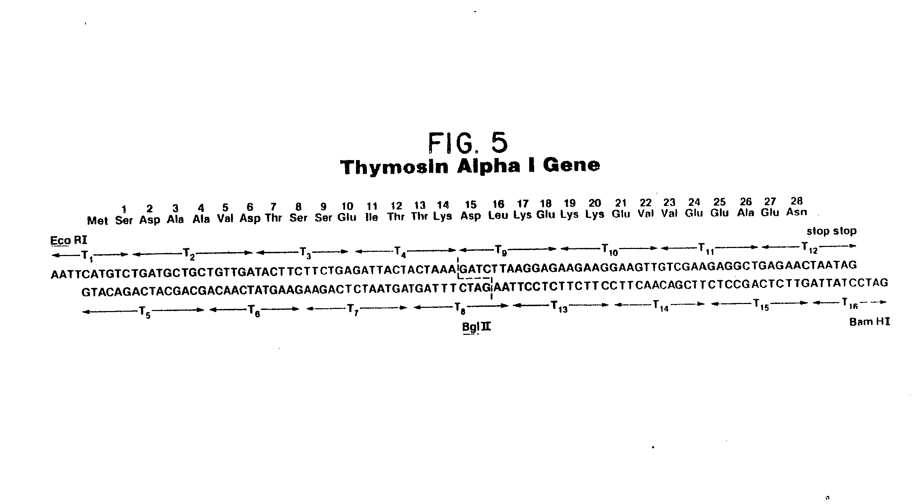

2. Construction of Plasmid poThal
Plasmid pThal was constructed by inserting a synthesized gene for thymosin alpha 1 into plasmid pBR322. The synthesis of the thymosin alpha 1 coding DNA involves the synthesis and subsequent ligation of the 16 oligonucleotides ($T_1$ through $T_{16}$) that are indicated by the double headed arrows in Figure 5 of the accompanying drawings. A Met codon ATG was inserted at the N-terminus and the 5' ends were designed with single-stranded cohesive termini to facilitate joining to plasmids cleaved with EcoR1 and BamH1. As can be readily appreciated, the Bg/II site in the center of the gene assists in the analysis of recombinant plasmids.

Oligodeoxyribonucleotides $T_1$ to $T_{16}$ were synthesized by the modified phosphotriester method using fully protected trideoxyribonucleotide building blocks (Itakura et al., 1977 and Crea et al., 1978). The various oligodeoxyribonucleotides are shown below in Table 1.

## Table 1

### SYNTHETIC OLIGONUCLEOTIDES FOR THYMOSINα1 GENE

| Compound | Sequence | Length | HPLC Analysis Retention Time (min)* |
|---|---|---|---|
| $T_1$ | A-A-T-T-C-A-T-G-T-C | 10 | 17.4 |
| $T_2$ | T-G-A-T-G-C-T-G-C-T-G-T-T-G-A | 15 | 24.3 |
| $T_3$ | T-A-C-T-T-C-T-TOC-T-G-A | 12 | 20.3 |
| $T_4$ | G-A-T-T-A-C-T-A-C-T-A-A-A | 13 | 22.0 |
| $T_5$ | G-C-A-G-C-A-T-C-A-G-A-C-A-T-G | 15 | 24.8 |
| $T_6$ | G-A-A-G-T-A-T-C-A-A-C-A | 12 | 20.1 |
| $T_7$ | A-G-T-A-A-T-C-T-C-A-G-A-A | 13 | 22.6 |
| $T_8$ | A-A-G-A-T-C-T-T-T-A-G-T | 12 | 20.2 |
| $T_9$ | G-A-T-C-T-T-A-A-G-G-A-G | 12 | 20.4 |
| $T_{10}$ | A-A-G-A-A-G-G-A-A-G-T-T | 12 | 21.1 |
| $T_{11}$ | G-T-C-G-A-A-G-A-G-G-C-T | 12 | 20.5 |
| $T_{12}$ | G-A-G-A-A-C-T-A-A-T-A-G | 12 | 20.4 |
| $T_{13}$ | C-T-T-C-T-T-C-T-C-C-T-T | 12 | 19.9 |
| $T_{14}$ | T-T-C-G-A-C-A-A-C-T-T-C | 12 | 20.5 |
| $T_{15}$ | G-T-T-C-T-C-A-G-C-C-T-C | 12 | 20.2 |
| $T_{16}$ | G-A-T-C-C-T-A-T-T-A | 10 | 17.2 |

*at ambient temperature

16

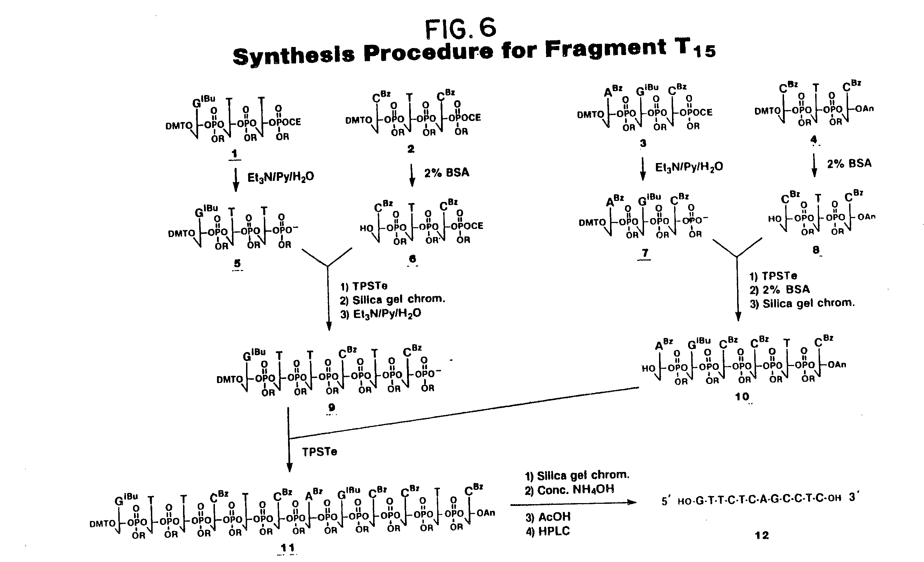

The above synthesis is typified by the following procedure for fragment $T_{15}$ as summarized in Figure 6 of the accompanying drawings. Various nucleotide fragments that are used in the synthesis of $T_{15}$ are numerically designated in the figure. The abbreviations employed are as follows: TPSTe, 2,4,6-triisopropyl-benzenesulfonyltetrazole; BSA, benzene sulfonic acid; TLC thin layer chromatography; HPLC, high performance liquid chromatography; DMT, 4,4'-dimethoxytrityl; CE, 2-cyanoethyl; R, p-chlorophenyl; Bz, benzoyl; An, anisoyl; iBu, isobutyryl; Py, pyridine; AcOH, acetic acid; $Et_3N$, trimethylamine.

The fully protected trideoxyribonucleotides 4 (85 mg, .05 mM) and 2 (180 mg, .1 mM) were deblocked at the 5' hydroxyls by treatment with 2% BSA in 7:3 (v/v) chloroform/methanol (10 and 20 ml, respectively) for 10 minutes at 0°C. Reactions were stopped by addition of saturated aqueous ammonium bicarbonate (2 ml), extracted with chloroform (25 ml) and washed with water (2 × 10 ml). The organic layers were dried (magnesium sulfate), concentrated to small volumes (about 5 ml) and precipitated by addition of petroleum ether (35°—60°C fraction). The colorless precipitates were collected by centrifugation and dried in a dessicator in vacuo to give 6 and 8 respectively, each homogeneous by silica gel tlc (Merck 60 F254, chloroform/methanol, 9:1).

Trimers 1 and 3 (270 mg, .15 mM; 145 mg, .075 mM) were converted into their phosphodiesters (5 and 7) by treatment with triethylamine/pyridine/water (1:3:1, v/v, 10 ml) for 25 minutes at ambient temperature. Reagents were removed by rotary evaporation and the residues dried by repeated evaporations with anhydrous pyridine (3 × 10 ml). Trimer 8 (.05 mM) and trimer 7 were combined with TPSTe (50 mg, .15 mM) in anhydrous pyridine (3 ml) and the reaction mixture left in vacuo at ambient temperature for two hours. TLC analysis showed that 95% of the trimer 8 had been converted into hexamer product (visualized by detection of the DMT group by spraying with 10% aqueous sulfuric acid and heating at 60°C). The reaction was quenched by addition of water (1 ml) and the solvent evaporated under reduced pressure. After removal of pyridine by coevaporations with toluene, the hexamer was deblocked at the 5' position with 2% BSA (8 ml) as described above for trimers 4 and 2. The product (10) was purified on a silica gel column (Merck 60 H, 3.5 × 5 cm) by step gradient elution with chloroform/methanol (98:2 to 95:5, v/v). Fractions containing product 10 were evaporated to dryness.

Similarly, trimer 5 was coupled to 6 and the fully protected product directly purified on silica gel. This latter compound was deblocked at the 3' end by triethylamine/pyridine/water as described above to give fragment 9.

Finally, hexamers 9 and 10 were coupled in anhydrous pyridine (2 ml) with TPSTe (75 mg, .225 mM) as the condensing agent. Upon completion (4 hours, ambient temperature) the mixture was rotary evaporated and the residue chromatographed on silica gel. Product 11 (160 mg) was obtained by precipitation with petroleum ether and appeared homogenous on TLC. A portion of compound 11 (20 mg) in pyridine (.5 ml) was completely deblocked by treatment with concentrated ammonium hydroxide (7 ml, 8 hours, 60°C) and subsequent treatment in 80% acetic acid (15 minutes, ambient temperature). After evaporation of acetic acid, the solid residue was dissolved in 4% aqueous ammonium hydroxide (v/v, 4 ml) and extracted with ethyl ether (3 × 2 ml). The aqueous phase was concentrated to 1—2 ml and a portion applied to HPLC for purification of 12. The fractions corresponding to the major peak were pooled (ca 2.0 $O.D._{254}$ units) and concentrated to about 5 ml. The final product 12 was desalted on Bio-gel P-2 (1.5 × 100 cm) by elution with 20% aqueous ethanol, reduced to dryness and resuspended in water (200 µl) to give a solution of $A_{254}$ = 10. The sequence of 12 was confirmed by two-dimensional sequence analysis.

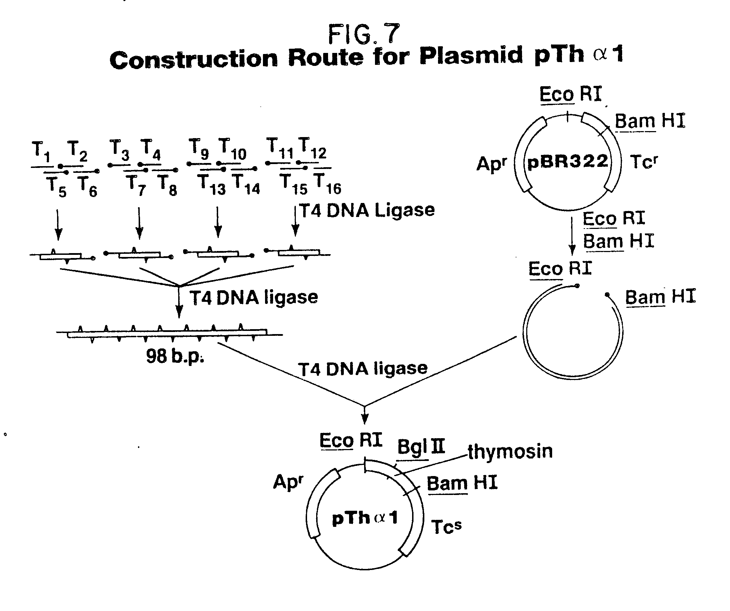

The complete thymosin alpha 1 gene was assembled from the 16 synthetic oligo-nucleotides by methods previously described in detail for somatostatin (Itakura et al., 1977) and growth hormone (Goeddel et al., 1979, Nature 281:544). Ten microgram quantities of oligonucleotides $T_2$ through $T_{15}$ were quantitatively phosphorylated with [$\gamma$-$^{32}$P]-ATP (New England Nuclear) in the presence of $T_4$ polynucleotide kinase (Goeddel et al., 1979), to give specific activities of approximately 1 Ci/mmol. Radiolabelled fragments were purified by 20% polyacrylamide/7 M urea gel electrophoresis and sequences of the eluted fragments were verified by two-dimensional electrophoresis/homochromatography (Jay et al., 1974, Nucleic Acids Res. 1:331) of partial snake venom digests. Fragments $T_1$ and $T_{16}$ were left unphosphorylated to minimize undesired polymerization during subsequent ligation reactions. These oligonucleotides (2 µg each) were assembled in four groups of four fragments (See figure 7 of the accompanying drawings), by $T_4$ DNA ligase using published procedures (Goeddel et al., 1979). The reaction products were purified by gel electrophoresis on a 15% polyacrylamide gel containing 7 M urea (Maxam and Gilbert, 1977, Proc. Nat. Acad. Sci. USA 71:3455). The four isolated products were ligated together and the reaction mixture resolved by 10% polyacrylamide gel electrophoresis. DNA in the size range of the thymosin alpha 1 gene (90—105 base pairs) was electroeluted.

Plasmid pBR322 (.5 µg) was treated with BamHI and EcoRI restriction endonucleases and the fragments separated by polyacrylamide gel electrophoresis. The large fragment was recovered from the gel by electroelution and subsequently ligated to the assembled synthetic DNA (Goeddel et al., 1979). This mixture was used to transform E. coli K12 strain 294, ATCC No. 31446. Five percent of the transformation mixture was plated on LB plates containing 20 µg/ml ampicillin. The ampicillin resistant colonies obtained were sensitive to tetracycline, suggesting insertion into the tetracycline resistance gene. Analysis of the plasmids from these colonies showed that in each case the plasmid, designated pThα1, contained (a) a Bg/II site not found in pBR322 itself, thus indicating the presence of the thymosin alpha 1 gene as shown in Figure 5, and (b) a fragment of approximately 105 base pairs generated by BamHI/EcoRI cleavage. The construction route for plasmid pThα1 (not drawn to scale), is presented in Figure 7 of the accompanying drawings wherein the heavy dots indicate 5'-phosphate groups.

3. Reaction of Treated pThα1 and LE'(d) Fragment

The plasmid pThα1 contains a gene specifying ampicillin resistance and a structural gene specifying thymosin alpha 1 cloned at its 5' coding strand end into an *Eco*RI site and at its 3' end into a *Bam*HI site. The thymosin gene contains a *Bgl*II site as well. To create a plasmid capable of accepting the LE'(d) fragment prepared above, pThα1 was *Eco*RI digested followed by Klenow polymerase I reaction with TTP and dATP to blunt the *Eco*RI residues. *Bgl*II digestion of the resulting product created a linear DNA fragment containing the gene for ampicillin resistance and, at its opposite ends, a sticky *Bgl*II residue and a blunt end. The resulting product could be recirculated by reaction with the LE'(d) fragment containing a *Bgl*II sticky end and a blunt end in the presence of T4 ligase to form the plasmid pTrp24. In doing so, an *Eco*RI site is recreated at the position where blunt end ligation occurred.

E. Construction of Plasmid pSOM7Δ2Δ4

Successive digestion of pTrp24 with *Bgl*II and *Eco*RI, followed by PAGE and electroelution, yields a fragment having codons for the LE'(d) polypeptide with a *Bgl*II sticky end and an *Eco*RI sticky end adjacent to its 3' coding terminus. The LE'(d) fragment can be cloned into the *Bgl*II site of plasmid pSom7Δ2 to form and LE' polypeptide/somatostatin fusion protein expressed under the control of the tryptophan promoter/operator. To do so requires (1) partial *Eco*RI digestion of pSom7Δ2 in order to cleave the *Eco*RI site distal to the tryptophan promoter/operator, and (2) proper choice of the primer sequence to properly maintain the codon reading frame and recreate an *Eco*RI cleavage site.

Thus, 16 μg of plasmid pSom7Δ2 was diluted into 200 μl of buffer containing 20 mM Tris, pH 7.5, 5 mM MgCl2, .02 NP40 detergent, and 100 mM NaCl and treated with .5 units *Eco*RI. After 15 minutes at 37°C, the reaction mixture was phenol extracted, chloroform extracted, ethanol precipitated, and subsequently digested with *Bgl*II. The larger resulting fragment was isolated by the PAGE procedure followed by electroelution. This fragment contains the codons "LE'(p)" for the proximal end of the LE' polypeptide, i.e., those upstream from the *Bgl*II site. This fragment was next ligated to the above LE'(d) fragment in the presence of T4 DNA ligase to form the plasmid pSom7Δ2Δ4, which upon transformation into *E. coli* strain 294, efficiently produced a fusion protein consisting of the fully reconstituted LE polypeptide and somatostatin under the control of the tryptophan promoter/operator.

F. Construction of Linear DNA Having a *Pst*I Residue at the 3' end and a *Bgl*II Residue at its 5' End Bounding a Gene Specifying Tetracycline Resistance

Plasmid pBR322 was *Hind*III digested and the protruding *Hind*III ends were digested with SI nuclease. The SI nuclease digestion involved treatment of 10 μg of *Hind*III-cleaved pBR322 in 30 μl SI buffer (.3M NaCl, 1 mM ZnCl2, 25 mM sodium acetate, pH 4.5) with 300 units SI nuclease for 30 minutes at 15°C. The reaction was stopped by the addition of 1 μl of 30 × SI nuclease stop solution (.8M tris base, 5 mM EDTA). The mixture was phenol extracted, chloroform extracted, ethanol precipitated, and then *Eco*RI digested as previously described. The resulting fragment, obtained by the PAGE procedure followed by electroelution, has an *Eco*RI sticky end and a blunt end whose coding strand begins with the nucleotide thymidine. The SI-digested *Hind*III residue beginning with thymidine can be joined to a Klenow Polymerase I-treated *Bgl*II residue so as to reconstitute the *Bgl*II restriction site upon ligation.

Therefore plasmid pSOM7Δ2, prepared in Example 7-1C, was *Bgl*II digested and the resulting *Bgl*II sticky ends were made double stranded by treatment with Klenow Polymerase I using all four deoxynuclectide triphosphates. *Eco*RI cleavage of the resulting product, followed by PAGE and electroelution of the small fragment, yielded a linear piece of DNA containing the tryptophan promoter/operator and codons of the LE' "proximal" sequence upstream from the *Bgl*II site ("LE'(p)"). The product had an *Eco*RI end and a blunt end resulting from filling in the *Bgl*II site. However, the *Bgl*II site is reconstituted by ligation of the blunt end to the blunt end of the above SI-digested *Hind*III fragment. Thus, the two fragments were ligated in the presence of T4 DNA ligase to form the recircularized plasmid pHKY10 which was propagated by transformation into competent *E. coli* strain 294 cells. Tetracycline resistant cells bearing the recombinant plasmid pHKY10 were selected and the plasmid DNA extracted. Digestion with *Bgl*II and *Pst*I, followed by isolation by the PAGE procedure and electroelution of the large fragment, yielded the desired linear piece of DNA having *Pst*I and *Bgl*II sticky ends. This DNA fragment, thus produced from pHKY10, contains the origin of replication and therefore is useful as a component in the construction of plasmid pIA7Δ4Δ1 in which both the genes coding for the trp LE' polypeptide fusion protein and the tetracycline resistance are controlled by the trp promoter/operator.

G. Construction of Linear DNA Having the Trp Promoter/Operator

Plasmid pSOM7Δ2Δ4, prepared in Example 7-1C, was subjected to partial *Eco*RI digestion followed by *Pst*I digestion. The resulting fragment contained the trp promoter/operator and was isolated by the PAGE procedure followed by electroelution. Partial *Eco*RI digestion was necessary to obtain a fragment which was cleaved adjacent to the 5' end of the somatostatin gene but not cleaved at the *Eco*RI site present between the ampicillin resistance gene and the trp promoter/operator. Ampicillin resistance lost by the *Pst*I

cut in the ampicillin resistance gene can be restored upon ligation with the final pHKY10 linear DNA derivative produced in Example 7-1F above.

### H. Isolation of the Insulin A Chain Structural Gene

The insulin A chain structural gene was obtained by the *Eco*RI and *Bam*HI digestion of plasmid pIA1, whose construction is disclosed in Goeddel *et al.*, 1979, Proc. Nat. Acad. Sci. USA 76:106. The plasmid can also be obtained from *E. coli* K12 strain 94/pIA1 (ATCC 31448). The desired fragment was purified by PAGE and electroelution and had *Eco*RI and *Bam*HI termini.

### I. Ligation of the Insulin A Chain Structural Gene, the Trp Promoter/Operator and the pHKY10 Linear DNA Fragment Having *Pst*I and *Bg*III Termini

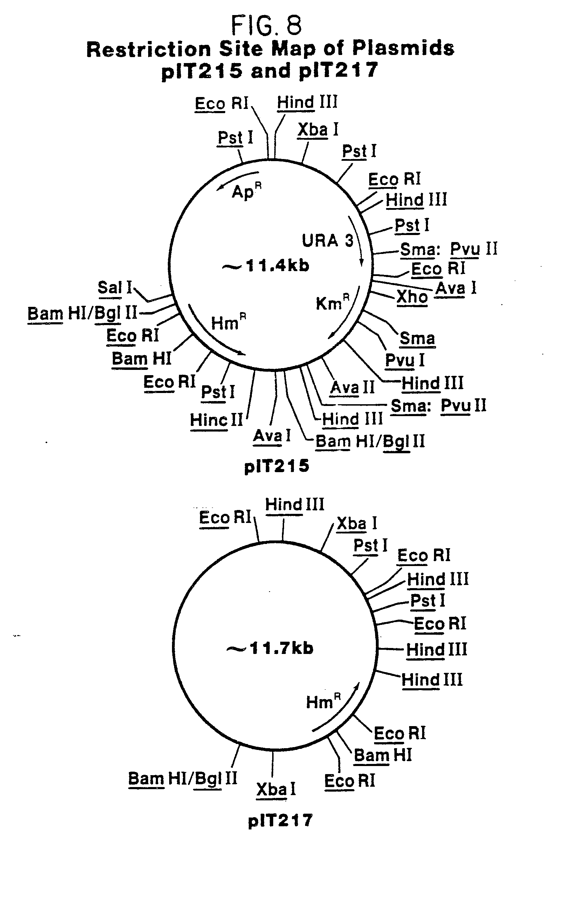

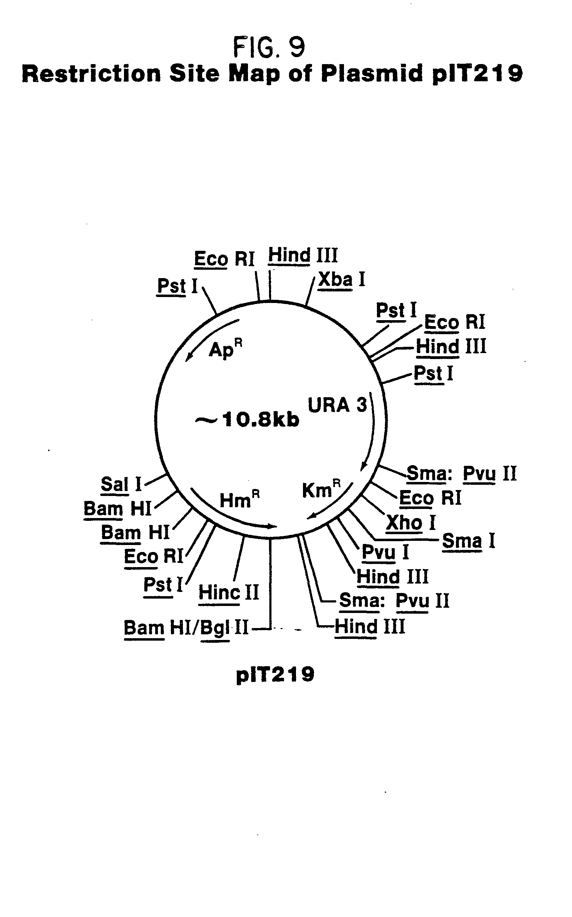

The Insulin A Chain structural gene, the linear DNA fragment containing the trp promoter/operator (prepared in Example 7-1G), and the pHKY10 linear DNA fragment (prepared in Example 7-1F), were ligated together in proper orientation, as depicted in Figure 8, to form the desired plasmid pIA7Δ4Δ1. Plasmid pIA7Δ4Δ1 can be readily selected because of the restoration of ampicillin and tetracycline resistance.

### 2. Ligation of the Plasmid pIT123 ~1.3 kb *Bam*HI-*Bg*III and Plasmid pIA7Δ4Δ1 ~4.5 kb *Bam*HI-*Bg*III Fragments

A. *Bam*HI-*Bg*III Digestion of Plasmid pIA7Δ4Δ1 and Isolation of the ~4.5 kb Fragment

The desired digestion and isolation were carried out in substantial accordance with the teaching of Example 2A except that plasmid pIA7Δ4Δ1 and *Bg*III and *Bam*HI restriction enzymes and manufacturer-recommended salts, rather than plasmid pIT123 and *Hph*I and *Pst*I restriction enzymes and salts, were used.

### B. Ligation and Transformation

The desired ligation and transformation was carried out in substantial accordance with the teaching of Example 3C excet that the ~4.5 kb *Bam*HI-*Bg*III fragment of plasmid pIA7Δ4Δ1 and *E. coli* K12 JA221 (NRRL B-15211), rather than plasmid pUC7 and *E. coli* K12 RR1ΔM15, were used. The transformed cells were plated onto TY plates containing 50 µg/ml ampicillin and subsequently patched onto plates containing 200 µg/ml hygromycin B. The desired hygromycin B resistant *E. coli* K12 JA221/pIT125 transformants were conventionally cultured for subsequent production and isolation of plasmid pIT125. Plasmid pIT125 can transform conventional *E. coli* strains such as, for example, *E. coli* K12, *E. coli* K12 RR1 and *E. coli* K12 HB101 in substantial accordance with the transformation teaching of Example 2B. A restriction site map of plasmid pIT125 is presented in Figure 4 of the accompanying drawings.

Additional illustrative plasmids and transformants constructed in accordance with the foregoing teachings are presented below in Tables 2 and 3.

TABLE 2

Representative Plasmids

| Example No. | Name | ~Size (in kb) | Markers | | Construction |
|---|---|---|---|---|---|
| | | | *E. coli* | Yeast | |
| 8 | pIT143 | 3.0 | Ap^R | | *Mbo*II digestion of the ~958 bp *Cla*I-*Hinc*II fragment of pIT141 followed by removal of the extensions, attachment of *Bam*HI linkers of the sequence TGGATCCA and ligation into *Bam*HI-digested plasmid pUC8 |
| 9 | pIT212 | 8.9 | Ap^R | URA3 | Ligation of ~1.3kb *Bam*HI-*Bg*/II fragment of pIT123 into *Bam*HI-digested pRB5 (ATCC No. 37051) |
| 10 | pIT213 | 10.6 | Ap^R Km^R | URA3 | Ligation of ~1.7 kb *Pvu*II fragment of plasmid pNG59* into *Sma*I-digested plasmid pIT212 |
| 11 | pIT215 | 11.4 | Ap^R Km^R | URA3 Hm^R | Ligation of ~750 bp *Bam*HI-*Bg*/II fragment of pIT118 into *Bam*HI-digested plasmid pIT213 in the orientation depicted in Figure 8. |
| 12 | pIT217 | 11.7 | Ap^R Km^R | URA3 Hm^R | Ligation of ~1 kb *Bam*HI-*Bg*/II fragment of pIT120 into *Bam*HI-digested plasmid pIT213 in the orientation depicted in Figure 8. |
| 13 | pIT219 | 10.8 | Ap^R Km^R | URA3 Hm^R | Ligation of the ~230 bp *Bam*HI fragment of plasmid pIT143 into *Bam*HI-digested plasmid pIT213 in the orientation depicted in Figure 9. |

\* Plasmid pNG59 can be obtained and conventionally isolated from *E. coli* K12 RR1/pNG59, a strain deposited and made part of the permanent stock culture collection of the Northern Regional Research Laboratory, Peoria, Illinois. The strain is available as a preferred source and stock reservoir of the plasmid under the accession number NRRL B—15604.

TABLE 3

Representative Transformants

*E. coli*/R
*E. coli* K12/R
*E. coli* K12 JA221/R
*E. coli* K12 HB101/R
*E. coli* K12 RR1/R

*Saccharomyces cerevisiae*/R[1]
wherein R is selected from the group consisting of plasmids pIT143, pIT212, pIT213, pIT215, pIT217 and pIT219 and wherein R[1] is selected from the group consisting of plasmids pIT212, pIT213, pIT215, pIT217 and pIT219.

# EP 0 135 291 B1

**Claims for the Contracting States: BE CH DE FR GB IT LI LU NL SE**

1. A DNA encoding the last 338, 339 or 340 amino acids of hygromycin B phosphotransferase.
2. The DNA of Claim 1 wherein the DNA comprises

```
Rm   R2n  CCT GAA CTC ACC GCG ACG TCT GTC GAG AAG
 R1m  R3n  GGA CTT GAG TGG CGC TGC AGA CAG CTC TTC

TTT CTG ATC GAA AAG TTC GAC AGC GTC TCC GAC CTG ATG
AAA GAC TAG CTT TTC AAG CTG TCG CAG AGG CTG GAC TAC

CAG CTC TCG GAG GGC GAA GAA TCT CGT GCT TTC AGC TTC
GTC GAG AGC CTC CCG CTT CTT AGA GCA CGA AAG TCG AAG

GAT GTA GGA GGG CGT GGA TAT GTC CTG CGG GTA AAT AGC
CTA CAT CCT CCC GCA CCT ATA CAG GAC GCC CAT TTA TCG

TGC GCC GAT GGT TTC TAC AAA GAT CGT TAT GTT TAT CGG
ACG CGG CTA CCA AAG ATG TTT CTA GCA ATA CAA ATA GCC

CAC TTT GCA TCG GCC GCG CTC CCG ATT CCG GAA GTG CTT
GTG AAA CGT AGC CGG CGC GAG GGC TAA GGC CTT CAC GAA

GAC ATT GGG GAA TTC AGC GAG AGC CTG ACC TAT TGC ATC
CTG TAA CCC CTT AAG TCG CTC TCG GAC TGG ATA ACG TAG

TCC CGC CGT GCA CAG GGT GTC ACG TTG CAA GAC CTG CCT
AGG GCG GCA CGT GTC CCA CAG TGC AAC GTT CTG GAC GGA

GAA ACC GAA CTG CCC GCT GTT CTG CAG CCG GTC GCG GAG
CTT TGG CTT GAC GGG CGA CAA GAC GTC GGC CAG CGC CTC

GCC ATG GAT GCG ATC GCT GCG GCC GAT CTT AGC CAG ACG
CGG TAC CTA CGC TAG CGA CGC CGG CTA GAA TCG GTC TGC

AGC GGG TTC GGC CCA TTC GGA CCG CAA GGA ATC GGT CAA
TCG CCC AAG CCG GGT AAG CCT GGC GTT CCT TAG CCA GTT

TAC ACT ACA TGG CGT GAT TTC ATA TGC GCG ATT GCT GAT
ATG TGA TGT ACC GCA CTA AAG TAT ACG CGC TAA CGA CTA

CCC CAT GTG TAT CAC TGG CAA ACT GTG ATG GAC GAC ACC
GGG GTA CAC ATA GTG ACC GTT TGA CAC TAC CTG CTG TGG

GTC AGT GCG TCC GTC GCG CAG GCT CTC GAT GAG CTG ATG
CAG TCA CGC AGG CAG CGC GTC CGA GAG CTA CTC GAC TAC

CTT TGG GCC GAG GAC TGC CCC GAA GTC CGG CAC CTC GTG
GAA ACC CGG CTC CTG ACG GGG CTT CAG GCC GTG GAG CAC

CAC GCG GAT TTC GGC TCC AAC AAT GTC CTG ACG GAC AAC
GTG CGC CTA AAG CCG AGG TTG TTA CAG GAC TGC CTG TTG

AAT GGC CGC ATA ACA GCG GTC ATT GAC TGG AGC GAG GCG
TTA CCG GCG TAT TGT CGC CAG TAA CTG ACC TCG CTC CGC
```

21

```
ATG TTC GGG GAT TCC CAA TAC GAG GTC GCC AAC ATC TTC
!!! !!! !!! !!! !!! !!! !!! !!! !!! !!! !!! !!! !!!
TAC AAG CCC CTA AGG GTT ATG CTC CAG CGG TTG TAG AAG

TTC TGG AGG CCG TGG TTG GCT TGT ATG GAG CAG CAG ACG
!!! !!! !!! !!! !!! !!! !!! !!! !!! !!! !!! !!! !!!
AAG ACC TCC GGC ACC AAC CGA ACA TAC CTC GTC GTC TGC

CGC TAC TTC GAG CGG AGG CAT CCG GAG CTT GCA GGA TCG
!!! !!! !!! !!! !!! !!! !!! !!! !!! !!! !!! !!! !!!
GCG ATG AAG CTC GCC TCC GTA GGC CTC GAA CGT CCT AGC

CCG CGG CTC CGG GCG TAT ATG CTC CGC ATT GGT CTT GAC
!!! !!! !!! !!! !!! !!! !!! !!! !!! !!! !!! !!! !!!
GGC GCC GAG GCC CGC ATA TAC GAG GCG TAA CCA GAA CTG

CAA CTC TAT CAG AGC TTG GTT GAC GGC AAT TTC GAT GAT
!!! !!! !!! !!! !!! !!! !!! !!! !!! !!! !!! !!! !!!
GTT GAG ATA GTC TCG AAC CAA CTG CCG TTA AAG CTA CTA

GCA GCT TGG GCG CAG GGT CGA TGC GAC GCA ATC GTC CGA
!!! !!! !!! !!! !!! !!! !!! !!! !!! !!! !!! !!! !!!
CGT CGA ACC CGC GTC CCA GCT ACG CTG CGT TAG CAG GCT

TCC GGA GCC GGG ACT GTC GGG CGT ACA CAA ATC GCC CGC
!!! !!! !!! !!! !!! !!! !!! !!! !!! !!! !!! !!! !!!
AGG CCT CGG CCC TGA CAG CCC GCA TGT GTT TAG CGG GCG

AGA AGC GCG GCC GTC TGG ACC GAT GGC TGT GTA GAA GTA
!!! !!! !!! !!! !!! !!! !!! !!! !!! !!! !!! !!! !!!
TCT TCG CGC CGG CAG ACC TGG CTA CCG ACA CAT CTT CAT

CTC GCC GAT AGT GGA AAC CGA CGC CCC AGC ACT CGT CCG
!!! !!! !!! !!! !!! !!! !!! !!! !!! !!! !!! !!! !!!
GAG CGG CTA TCA CCT TTG GCT GCG GGG TCG TGA GCA GGC

AGG GCA AAG GAA R⁴
!!! !!! !!! !!! !⁵
TCC CGT TTC CTT R⁵
```

wherein
  A is deoxyadenyl,
  G is deoxyguanidyl,
  C is deoxycytidyl and
  T is thymidyl
  R and $R^2$ are deoxyribonucleotide triplets that independently encode lysine,
  $R^1$ and $R^3$ are deoxyribonucleotide triplets wherein the nitrogeneous bases are complementary to the respective and corresponding bases of R and $R^2$,
  m and n = 0 or 1, subject to the limitation that when n = 0, then m = 0 and when m = 1, then n = 1,
  $R^4$ is a deoxyribonucleotide triplet that encodes a translational stop codon and
  $R^5$ is a deoxyribonucleotide triplet wherein the nitrogenous based are complementary to the corresponding bases of $R^4$, subject to the limitation that the DNA is not associated with the transcriptional and translational activator sequence of plasmid pKC203 as obtainable from *E. coli* JR225 deposited with ATCC under accession number ATCC 31912.

3. The DNA of Claim 2 which is in translational reading phase with a transcriptional and translational activator sequence, subject to the limitation that the sequence is not identical to that encoded by plasmid pKC203.

4. The DNA of Claim 3 which comprises up to the first 15 amino-terminal codons of a structural gene homologous to the transcriptional and translational activator sequence.

5. The DNA of Claim 4 wherein $R^4$ is TAG and $R^5$ is ATC.

6. The DNA of any of Claims 1 to 5 wherein the gene homologous to the transcriptional and translational activator sequence is a bacterial gene.

7. The DNA of Claim 6 wherein the gene encodes up to 15 amino acids.

8. The DNA of Claim 6 or 7 wherein the gene is a portion of the *E. coli* lac Z gene.

9. The DNA of any of Claims 1 to 5 wherein the gene is a eukaryotic gene.

10. The DNA of Claim 9 wherein the gene is a portion of the yeast heat shock gene.

11. The DNA of any of Claims 2 to 10 wherein n = 1 and m = 0.

12. The DNA of any of Claims 2 to 10 wherein m and n = 1.

13. The DNA of any of Claims 2 to 10 wherein m and n = 0.

14. The DNA of Claim 13 which encodes the amino acid sequence

PRO GLU LEU THR ALA THR SER VAL GLU LYS

PHE LEU ILE GLU LYS PHE ASP SER VAL SER ASP LEU MET

GLN LEU SER GLU GLY GLU GLU SER ARG ALA PHE SER PHE

ASP VAL GLY GLY ARG GLY TYR VAL LEU ARG VAL ASN SER

CYS ALA ASP GLY PHE TYR LYS ASP ARG TYR VAL TYR ARG

HIS PHE ALA SER ALA ALA LEU PRO ILE PRO GLU VAL LEU

ASP ILE GLY GLU PHE SER GLU SER LEU THR TYR CYS ILE

SER ARG ARG ALA GLN GLY VAL THR LEU GLN ASP LEU PRO

GLU THR GLU LEU PRO ALA VAL LEU GLN PRO VAL ALA GLU

ALA MET ASP ALA ILE ALA ALA ALA ASP LEU SER GLN THR

SER GLY PHE GLY PRO PHE GLY PRO GLN GLY ILE GLY GLN

TYR THR THR TRP ARG ASP PHE ILE CYS ALA ILE ALA ASP

PRO HIS VAL TYR HIS TRP GLN THR VAL MET ASP ASP THR

VAL SER ALA SER VAL ALA GLN ALA LEU ASP GLU LEU MET

LEU TRP ALA GLU ASP CYS PRO GLU VAL ARG HIS LEU VAL

HIS ALA ASP PHE GLY SER ASN ASN VAL LEU THR ASP ASN

GLY ARG ILE THR ALA VAL ILE ASP TRP SER GLU ALA MET

PHE GLY ASP SER GLN TYR GLU VAL ALA ASN ILE PHE PHE

TRP ARG PRO TRP LEU ALA CYS MET GLU GLN GLN THR ARG

TYR PHE GLU ARG ARG HIS PRO GLU LEU ALA GLY SER PRO

ARG LEU ARG ALA TYR MET LEU ARG ILE GLY LEU ASP GLN

LEU TYR GLN SER LEU VAL ASP GLY ASN PHE ASP ASP ALA

ALA TRP ALA GLN GLY ARG CYS ASP ALA ILE VAL ARG SER

GLY ALA GLY THR VAL GLY ARG THR GLN ILE ALA ARG ARG

SER ALA ALA VAL TRP THR ASP GLY CYS VAL GLU VAL LEU

ALA ASP SER GLY ASN ARG ARG PRO SER THR ARG PRO ARG

ALA LYS GLU

wherein

MET is methionine,
LYS is lysine,
PRO is proline,
GLU is glutamic acid,
LEU is leucine,
THR is threonine,
ALA is alanine,
SER is serine,
VAL is valine,
PHE is phenylalanine,
ILE is isoleucine,
GLY is glycine,
ASP is aspartic acid,
GLN is glutamine,
ARG is arginine,
CYS is cysteine,
TRP is tryptophan,
ASN is asparagine,
HIS is histidine and
TYR is tyrosine.

15. Plasmid pIT123 which is shown in Figure 1 and constructed by the steps of (a) ligating ~2.75 kb *Sal*I-*Bgl*II fragment of plasmid pKC203 (ATCC Deposit No. 31912) and the ~4.1 kb *Sal*I-*Bgl*II fragment of plasmid pKC7 (ATCC Deposit No. 37084) to form plasmid pKC222; (b) providing the 325 bp *Hph*I-*Pst*I fragment of plasmid pKC222 with a *Bam*HI linker and an *Eco*RI linker; (c) ligating the linker provided fragment of step (b) with the *Eco*RI-*Bam*HI digest of known plasmid pBR322 to provide plasmid pIT122; and ligating the ~1.45 kb *Eco*RI fragment of plasmid pKC222 and the *Eco*RI digest of plasmid pIT122.

16. The DNA of Claim 2 which is the ~1.3 kb *Bam*HI-*Bgl*II restriction fragment of plasmid pIT123 of Claim 15.

17. A recombinant DNA cloning vector comprising the DNA of any of Claims 1 to 14 or Claim 16.

18. The vector of Claim 17 which is a plasmid.

19. Plasmid pIT144 which is constructed by ligating the ~1.3 kB *Bam*HI-*Bgl*II fragment of plasmid pIT123 of Claim 15 and the *Bam*HI digest of known plasmid pUC7.

20. Plasmid pIT207 which is shown in Figure 3 and constructed by ligating the ~750 bp *Bam*HI-*Bgl*II fragment of plasmid pIT118 (NRRL deposit No. B-15441) and the 16 kb *Bam*HI digest of plasmid pMC1587 (NRRL deposit No. B-15442).

21. Plasmid pIT208 which is shown in Figure 3 and constructed by ligating the ~1.3 kB *Bam*HI-*Bgl*II fragment of plasmid pIT123 of Claim 15 and the *Bam*HI digest of plasmid pIT207 of Claim 20.

22. The vector of Claim 18 which is

| Plasmid | ~ Size (in kb) | Constructed by |
|---|---|---|
| pIT212 | . 8.9 | Ligation of the ~1.3 kb *Bam*HI-*Bgl*II fragment of plasmid pIT123 of Claim 15 into the unique *Bam*HI restriction site of plasmid pRB5 (ATCC deposit No. 37051) |
| pIT213 | 10.6 | Blunt end ligation of ~1.7 kb *pVU*II fragment of plasmid pNG59* (NRRL deposit No. B-15604) into the unique *Sma*I restriction site of plasmid pIT212 |
| pIT215 | 11.4 | Ligation of ~750 bp *Bam*HI-*Bgl*II fragment of plasmid pIT118 (NRRL deposit No. B-15441) into the unique *Bam*HI restriction site of plasmid pIT213 in the orientation depicted in Figure 8. |
| pIT217 | 11.7 | Ligation of the ~1 kb *Bam*HI-*Bgl*II fragment of plasmid pIT120 (NRRL deposit No. B-15603) into the unique *Bam*HI restriction site of plasmid pIT213 in the orientation depicted in Figure 8. |
| pIT219 | 10.8 | Ligation of the ~230 bp *Bam*HI fragment of plasmid pIT143 into the Unique *Bam*HI restriction site of plasmid pIT213 in the orientation depicted in Figure 9, said plasmid pIT143 being constructed by digesting the 958 bp *Cla*I-*Hinc*II fragment of plasmid pIT141 (conventionally isolated from *E. coli* K12 JA221/pIT141, NRRL deposit No. B-15602) with the restriction enzyme *Mbo*II, removing the resultant extensions with the Klenow fragment of DNA polymerase, attaching *Bam*HI linkers with the sequence TGGATCCA and then ligating the linker-containing fragment into *Bam*HI-digested plasmid pUC8. |

24

23. A transformant comprising the recombinant DNA cloning vector of Claim 15, 18, 21 or 22.

24. The transformant of Claim 23 which is *E. coli*.

25. The transformant of Claim 23 which is *Saccharomyces cerevisiae.*

26. A transformant which is an *E. coli* host cell comprising the plasmid of Claim 20.

**Claims for the Contracting State: AT**

1. A process for preparing a plasmid comprising a DNA sequence encoding the last 338 amino acids of hygromycin B phosphotransferase, either alone or in translational reading phase with a transcriptional and translational activator sequence-containing gene or portion of a gene, which comprises the steps of (a) ligating the ~2.75 kb *Sal*I-*Bgl*II fragment of plasmid pKC203 (ATCC Deposit No. 31912) and the ~4.1 kb *Sal*I-*Bgl*II fragment of plasmid pKC7 (ATCC Deposit No. 37084) to form plasmid pKC222; (b) providing the 325 bp *Hph*I-*Pst*I fragment of plasmid pKC222 with a *Bam*HI linker and an *Eco*RI linker; (c) ligating the linker provided fragment of step (b) with the *Eco*RI-*Bam*HI digest of known plasmid pBR322 to provide plasmid pIT122; and (d) ligating the ~1.45 kb *Eco*RI fragment of plasmid pKC222 and the *Eco*RI digest of plasmid pIT122, to obtain plasmid pIT123 shown in Figure 1.

2. The process of claim 1 for preparing plasmid pIT144 which comprises ligating the ~1.3 kb *Bam*HI-*Bgl*II restriction fragment of plasmid pIT123 of claim 1 into *Bam*HI-digested plasmid pUC7.

3. The process of claim 1 for preparing plasmid pIT212 which comprises ligating the ~1.3 kb *Bam*HI-*Bgl*II restriction fragment of plasmid pIT123 of claim 1 into *Bam*HI-digested plasmid pRB5 (ATCC deposit No. 37051).

4. The process of claim 3 for preparing plasmid pIT213 which comprises ligating the ~1.7 kb *Pvu*II restriction fragment of plasmid pNC59* (NRRL deposit No. B-15604) into *Sma*I-digested plasmid pIT212 of claim 3.

5. The process of claim 4 for preparing plasmid pIT215 which comprises ligating the ~750 bp *Bam*HI-*Bgl*II restriction fragment of plasmid pIT118 (NRRL deposit No. B-15441) into *Bam*HI-digested plasmid pIT213 of claim 4.

6. The process of claim 4 for preparing plasmid pIT217 which comprises ligating the ~1 kb *Bam*HI-*Bgl*II restriction fragment of plasmid pIT120 (NRRL deposit No. B-15603) into *Bam*Hi-digested plasmid pIT213 of claim 4 in the orientation depicted in Figure 8.

7. The process of claim 1 for preparing plasmid pKC307 which comprises ligating *Hph*I-digested plasmid pIT104 and *Hinc*II-digested plasmid pUC8, plasmid pIT104 being constructed by providing *Sac*I-cut plasmid pKC222 of claim 1 with *Bam*HI linkers, digesting with *Bam*HI restriction enzyme, and ligating the resulting fragment with *Eco*RI-*Bam*HI digest of known plasmid pBR322.

8. The process of claim 7 for preparing plasmid pKC308 which comprises deleting the ~1.7 kb *Hind*III fragment from plasmid pKC307.

9. The process for preparing plasmid pIT207, shown in Figure 3, which comprises ligating the ~750 bp *Bam*HI-*Bgl*II fragment of plasmid pIT118 (NRRL deposit No. B-15441) into *Bam*HI-digested plasmid pMC1587 (NRRL deposit No. B-15442).

10. The process of claim 1 for preparing plasmid pIT208 which comprises ligating the ~1.3 kb *Bam*HI-*Bgl*II restriction fragment of plasmid pIT123 of claim 1 into *Bam*HI-digested plasmid pIT207 of claim 9.

11. The process for preparing plasmid pIT143 which comprises digesting the 958 bp *Cla*I-*Hinc*II fragment of plasmid pIT141 (NRRL deposit No. B-15602) with the restriction enzyme *Mbo*II, removing the resultant extensions attaching *Bam*HI linkers, and ligating the linker-containing fragment into *Bam*HI-digested plasmid pUC8.

12. The process of claim 4 for preparing plasmid pIT219 which comprises ligating the ~230 bp *Bam*HI fragment of plasmid pIT143 of claim 11 into *Bam*HI-digested plasmid pIT2313 of claim 4.

13. A plasmid prepared in accordance with the process of any of claims 1 to 12 present in an *E. coli* host cell.

14. A plasmid prepared in accordance with any of claims 1 to 12 present in a *Saccharomyces cerevisiae* host cell.

**Patentansprüche für die Vertragsstaaten: BE CH DE FR GB IT LI LU NL SE**

1. DNA— kodierend für die letzten 338, 339 oder 340 Aminosäuren von Hygromycin B-phosphotransferase.

DNA nach Anspruch 1, worin die DNA folgendes umfaßt:

```
Rm   R²n  CCT GAA CTC ACC GCG ACG TCT GTC GAG AAG
 ↓1   ↓3  ↓↓↓ ↓↓↓ ↓↓↓ ↓↓↓ ↓↓↓ ↓↓↓ ↓↓↓ ↓↓↓ ↓↓↓ ↓↓↓
R¹m  R³n  GGA CTT GAG TGG CGC TGC AGA CAG CTC TTC

TTT CTG ATC GAA AAG TTC GAC AGC GTC TCC GAC CTG ATG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
AAA GAC TAG CTT TTC AAG CTG TCG CAG AGG CTG GAC TAC

CAG CTC TCG GAG GGC GAA GAA TCT CGT GCT TTC AGC TTC
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
GTC GAG AGC CTC CCG CTT CTT AGA GCA CGA AAG TCG AAG

GAT GTA GGA GGG CGT GGA TAT GTC CTG CGG GTA AAT AGC
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
CTA CAT CCT CCC GCA CCT ATA CAG GAC GCC CAT TTA TCG

TGC GCC GAT GGT TTC TAC AAA GAT CGT TAT GTT TAT CGG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
ACG CGG CTA CCA AAG ATG TTT CTA GCA ATA CAA ATA GCC

CAC TTT GCA TCG GCC GCG CTC CCG ATT CCG GAA GTG CTT
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
GTG AAA CGT AGC CGG CGC GAG GGC TAA GGC CTT CAC GAA

GAC ATT GGG GAA TTC AGC GAG AGC CTG ACC TAT TGC ATC
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
CTG TAA CCC CTT AAG TCG CTC TCG GAC TGG ATA ACG TAG

TCC CGC CGT GCA CAG GGT GTC ACG TTG CAA GAC CTG CCT
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
AGG GCG GCA CGT GTC CCA CAG TGC AAC GTT CTG GAC GGA

GAA ACC GAA CTG CCC GCT GTT CTG CAG CCG GTC GCG GAG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
CTT TGG CTT GAC GGG CGA CAA GAC GTC GGC CAG CGC CTC

GCC ATG GAT GCG ATC GCT GCG GCC GAT CTT AGC CAG ACG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
CGG TAC CTA CGC TAG CGA CGC CGG CTA GAA TCG GTC TGC

AGC GGG TTC GGC CCA TTC GGA CCG CAA GGA ATC GGT CAA
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
TCG CCC AAG CCG GGT AAG CCT GGC GTT CCT TAG CCA GTT

TAC ACT ACA TGG CGT GAT TTC ATA TGC GCG ATT GCT GAT
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
ATG TGA TGT ACC GCA CTA AAG TAT ACG CGC TAA CGA CTA

CCC CAT GTG TAT CAC TGG CAA ACT GTG ATG GAC GAC ACC
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
GGG GTA CAC ATA GTG ACC GTT TGA CAC TAC CTG CTG TGG

GTC AGT GCG TCC GTC GCG CAG GCT CTC GAT GAG CTG ATG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
CAG TCA CGC AGG CAG CGC GTC CGA GAG CTA CTC GAC TAC

CTT TGG GCC GAG GAC TGC CCC GAA GTC CGG CAC CTC GTG
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
GAA ACC CGG CTC CTG ACG GGG CTT CAG GCC GTG GAG CAC

CAC GCG GAT TTC GGC TCC AAC AAT GTC CTG ACG GAC A̶A̶C̶
↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓ ↓↓↓
GTG CGC CTA AAG CCG AGG TTG TTA CAG GAC TGC CTG T̶T̶G̶
```

```
AAT GGC CGC ATA ACA GCG GTC ATT GAC TGG AGC GAG GCG
!!! !!! !!! !!! !!! !!! !!! !!! !!!:!!! !!! !!! !!!
TTA CCG GCG TAT TGT CGC CAG TAA CTG ACC TCG CTC CGC

ATG TTC GGG GAT TCC CAA TAC GAG GTC GCC AAC ATC TTC
!!! !!! !!! !!! !!! !!! !!! !!! !!! !!! !!! !!! !!!
TAC AAG CCC CTA AGG GTT ATG CTC CAG CGG TTG TAG AAG

TTC TGG AGG CCG TGG TTG GCT TGT ATG GAG CAG CAG ACG
!!! !!! !!! !!! !!! !!! !!! !!! !!! !!! !!! !!! !!!
AAG ACC TCC GGC ACC AAC CGA ACA TAC CTC GTC GTC TGC

CGC TAC TTC GAG CGG AGG CAT CCG GAG CTT GCA GGA TCG
!!! !!! !!! !!! !!! !!! !!! !!! !!! !!! !!! !!! !!!
GCG ATG AAG CTC GCC TCC GTA GGC CTC GAA CGT CCT AGC

CCG CGG CTC CGG GCG TAT ATG CTC CGC ATT GGT CTT GAC
!!! !!! !!! !!! !!! !!! !!! !!! !!! !!! !!! !!! !!!
GGC GCC GAG GCC CGC ATA TAC GAG GCG TAA CCA GAA CTG

CAA CTC TAT CAG AGC TTG GTT GAC GGC AAT TTC GAT GAT
!!! !!! !!! !!! !!! !!! !!! !!! !!! !!! !!! !!! !!!
GTT GAG ATA GTC TCG AAC CAA CTG CCG TTA AAG CTA CTA

GCA GCT TGG GCG CAG GGT CGA TGC GAC GCA ATC GTC CGA
!!! !!! !!! !!! !!! !!! !!! !!! !!! !!! !!! !!! !!!
CGT CGA ACC CGC GTC CCA GCT ACG CTG CGT TAG CAG GCT

TCC GGA GCC GGG ACT GTC GGG CGT ACA CAA ATC GCC CGC
!!! !!! !!! !!! !!! !!! !!! !!! !!! !!! !!! !!! !!!
AGG CCT CGG CCC TGA CAG CCC GCA TGT GTT TAG CGG GCG

AGA AGC GCG GCC GTC TGG ACC GAT GGC TGT GTA GAA GTA
!!! !!! !!! !!! !!! !!! !!! !!! !!! !!! !!! !!! !!!
TCT TCG CGC CGG CAG ACC TGG CTA CCG ACA CAT CTT CAT

CTC GCC GAT AGT GGA AAC CGA CGC CCC AGC ACT CGT CCG
!!! !!! !!! !!! !!! !!! !.!! !!! !!! !!! !!! !!! !!!
GAG CGG CTA TCA CCT TTG GCT GCG GGG TCG TGA GCA GGC
```

$$AGG\ GCA\ AAG\ GAA\ R^4$$
$$!!!\ !!!\ !!!\ !!!\ R^5$$
$$TCC\ CGT\ TTC\ CTT$$

worin

A Desoxyadenyl bedeutet,

G Desoxyguanidyl bedeutet,

C Desoxycytidyl bedeutet und

T Thymidyl bedeutet,

R und $R^2$ Desoxyribonucleotid-Tripplets bedeuten, die unabhängig für Lysin kodieren,

$R^1$ und $R^3$ Desoxyribonucleotid-Tripplets bedeuten, worin die Stickstoffbasen komplementär zu den jeweiligen und entsprechenden Basen von R und $R^2$ sind,

m und n den Wert 0 oder 1 haben, mit der Maßgabe, daß wenn n den Wert 0 hat, m 0 ist und daß, wenn m den Wert 1 hat, n 1 ist,

$R^4$ ein Desoxyribonucleotid-Tripplet bedeutet, das für ein translationales Stoppcodon kodiert und

$R^5$ ein Desoxyribonucleotid-Tripplet bedeutet, worin die Stickstoffbasen komplementär zu den entsprechenden Basen von $R^4$ sind,

mit der Maßgabe, daß die DNA nicht mit der transkriptionalen und translationalen Aktivatorsequenz von Plasmid pKC203, erhältlich aus E. coli JR225, hinterlegt bei ATCC unter der Hinterlegungs-Nr. ATCC 31912, assoziiert ist.

3. DNA nach Anspruch 2, die sich in translationaler Lesephase mit einer transkriptionalen und translationalen Aktivatorsequenz befindet, mit der Maßgabe, daß die Sequenz nicht mit der vom Plasmid pKC203 kodierten Sequenz identisch ist.

4. DNA nach Anspruch 3, die bis zu 15 der ersten 15 aminoterminalen Codons eines Strukturgens, das zur transkriptionalen und translationalen Aktivatorsequenz homolog ist, enthält.

5. DNA nach Anspruch 4, worin $R^4$ TAG bedeutet und $R^5$ ATC bedeutet.

6. DNA nach einem der Ansprüche 1 bis 5, worin das zur transkriptionalen und translationalen Aktivatorsequenz homologe Gen ein bakterielles Gen ist.

7. DNA nach Anspruch 6, worin das Gen für bis zu 15 Aminosäuren kodiert.

8. DNA nach Anspruch 6 oder 7, worin das Gen ein Teil des E. coli lac Z-Gens ist.

27

## EP 0 135 291 B1

9. DNA nach einem der Ansprüche 1 bis 5, worin das Gen ein eukaryontisches Gen ist.
10. DNA nach Anspruch 9, worin das Gen ein Teil des Hefe-Wärmeschock-Gens ist.
11. DNA nach einem der Ansprüche 2 bis 10, worin n den Wert 1 und m den Wert 0 hat.
12. DNA nach einem der Ansprüche 2 bis 10, worin m und n den Wert 1 haben.
13. DNA nach einem der Ansprüche 2 bis 10, worin m und n den Wert 0 haben.
14. DNA nach Anspruch 13, die für folgende Aminosäuresequenz kodiert:

```
PRO GLU LEU THR ALA THR SER VAL GLU LYS

PHE LEU ILE GLU LYS PHE ASP SER VAL SER ASP LEU MET

GLN LEU SER GLU GLY GLU GLU SER ARG ALA PHE SER PHE

ASP VAL GLY GLY ARG GLY TYR VAL LEU ARG VAL ASN SER

CYS ALA ASP GLY PHE TYR LYS ASP ARG TYR VAL TYR ARG

HIS PHE ALA SER ALA ALA LEU PRO ILE PRO GLU VAL LEU

ASP ILE GLY GLU PHE SER GLU SER LEU THR TYR CYS ILE

SER ARG ARG ALA GLN GLY VAL THR LEU GLN ASP LEU PRO

GLU THR GLU LEU PRO ALA VAL LEU GLN PRO VAL ALA GLU

ALA MET ASP ALA ILE ALA ALA ALA ASP LEU SER GLN THR

SER GLY PHE GLY PRO PHE GLY PRO GLN GLY ILE GLY GLN

TYR THR THR TRP ARG ASP PHE ILE CYS ALA ILE ALA ASP

PRO HIS VAL TYR HIS TRP GLN THR VAL MET ASP ASP THR

VAL SER ALA SER VAL ALA GLN ALA LEU ASP GLU LEU MET

LEU TRP ALA GLU ASP CYS PRO GLU VAL ARG HIS LEU VAL

HIS ALA ASP PHE GLY SER ASN ASN VAL LEU THR ASP ASN

GLY ARG ILE THR ALA VAL ILE ASP TRP SER GLU ALA MET

PHE GLY ASP SER GLN TYR GLU VAL ALA ASN ILE PHE PHE

TRP ARG PRO TRP LEU ALA CYS MET GLU GLN GLN THR ARG

TYR PHE GLU ARG ARG HIS PRO GLU LEU ALA GLY SER PRO

ARG LEU ARG ALA TYR MET LEU ARG ILE GLY LEU ASP GLN

LEU TYR GLN SER LEU VAL ASP GLY ASN PHE ASP ASP ALA

ALA TRP ALA GLN GLY ARG CYS ASP ALA ILE VAL ARG SER

GLY ALA GLY THR VAL GLY ARG THR GLN ILE ALA ARG ARG

SER ALA ALA VAL TRP THR ASP GLY CYS VAL GLU VAL LEU

ALA ASP SER GLY ASN ARG ARG PRO SER THR ARG PRO ARG

ALA LYS GLU
```

28

worin

MET Methionin bedeutet,

LYS Lysin bedeutet,

PRO Prolin bedeutet,

GLU Glutaminsäure bedeutet,

LEU Leucin bedeutet,

THR Threonin bedeutet,

ALA Alanin bedeutet,

SER Serin bedeutet,

VAL Valin bedeutet,

PHE Phenylalanin bedeutet,

ILE Isoleucin bedeutet,

GLY Glycin bedeutet,

ASP Asparaginsäure bedeutet,

GLN Glutamin bedeutet,

ARG Arginin bedeutet,

CYS Cystein bedeutet,

TRP Tryptophan bedeutet,

ASN Asparagin bedeutet,

HIS Histidin bedeutet und

TYR Tyrosin bedeutet.

15. Plasmid pIT123, das in Fig. 1 dargestellt ist, und durch folgende Stufen konstruiert ist: (a) Ligation des ~2,75 kb-SalI-BglII-Fragments des Plasmids pKC203 (ATCC Hinterlegungs-Nr. 31912) und des ~4,1 kb SalIBglII-Fragments des Plasmid pKC7 (ATCC Hinterlegungs-Nr. 37084) unter Bildung des Plasmids pKC222; (b) Versehen des 325 bp-HphI-PstI-Fragments von Plasmid pKC222 mit einem BamHI-Linker und einem EcoRI-Linker; (c) Ligation des mit einem Linker versehenen Fragments von Stufe (b) mit dem EcoRI-BamHI-Verdauungsprodukt des bekannten Plasmids pBR322 zur Bildung des Plasmids pIT122; und Ligation des ~1,45 kb-EcoRI-Fragments von Plasmid pKC222 und des EcoRI-Verdauungsprodukts von Plasmid pIT122.

16. DNA nach Anspruch 2, bei der es sich um das ~1,3 kb-BamHI-BglII-Restriktionsfragment von Plasmid pIT123 von Anspruch 15 handelt.

17. Rekombinater DNA-Klonierungsvektor, enthaltend DNA von einem der Ansprüche 1 bis 14 oder 16.

18. Vektor nach Anspruch 17, bei dem es sich um ein Plasmid handelt.

19. Plasmid pIT144, das konstruiert ist durch Ligation des ~1,3 kb-BamHI-BglII-Fragments des Plasmids pIT123 nach Anspruch 15 und des BamHI-Verdauungsprodukts des bekannten Plasmids pUC7.

20. Plasmid pIT207, das in Fig. 3 dargestellt ist und konstruiert ist durch Ligation des ~750 bp-BamHI-BglII-Fragments des Plasmids pIT118 (NRRL-Hinterlegungs-Nr. B—15441) und des 16 kb BamHI-Verdauungsprodukts des Plasmids pMC 1587 (NRRL-Hinterlegungs-Nr. B—15442.

21. Plasmid pIT208, das in Fig. 3 dargestellt ist und konstruiert ist durch Ligation des ~1,3 kb-BamHI-BglII-Fragments des Plasmids pIT123 nach Anspruch 15 und des BamHI-Verdauungsprodukts des Plasmids pIT207 nach Anspruch 20.

22. Vektor nach Anspruch 18, bei dem es sich um folgendes Produkt handelt:

| Plasmid | ~Größe (in kb) | Konstruiert durch |
|---------|----------------|-------------------|
| pIT212 | 8,9 | Ligation des ~1,3 kb-BamHI-BglII-Fragments des Plasmids pIT123 in die einzige BamHI-Restriktionsstelle des Plasmids pRB5 (Hinterlegungs-Nr. 37051) |
| pIT213 | 10,6 | Stumpfendige Verknüfung des ~1,7 kb-pVUII-Fragments von Plasmid pNG59* (NRRL-Hinterlegungs-Nr. B—15604) in die einzige SmaI-Restriktionsstelle von Plasmid pIT212 |
| pIT215 | 11,4 | Ligation des ~750 bp-BamHI-BglII-Fragments des Plasmids pIT118 (NRRL-Hinterlegungs-Nr. B—15441) in die einzige BamHI-Restriktionsstelle des Plasmids pIT213 in der in Fig. 8 dargestellten Orientierung. |
| pIT217 | 11,7 | Ligation des ~1 kb-BamHI-BglII-Fragments des Plasmids pIT120 (NRRL-Hinterlegungs-Nr. B—15603) in die einzige BamHI-Restriktionsstelle des Plasmids pIT213 in der in Fig. 8 dargestellten Orientierung |

29

| Plasmid | ~Größe (in kb) | Konstruiert durch |
|---|---|---|
| pIT219 | 10,8 | Ligation des ~230 bp-BamHI-Fragments des Plasmids pIT143 in die einzige BamHI-Restriktionsstelle des Plasmids pIT213 in der in Fig. 9 dargestellten Orientierung, wobei das Plasmid pIT143 konstruiert ist durch Verdauung des 958 bp-ClaI-HincII-Fragments des Plasmids pIT141 (in herkömmlicher Weise isoliert aus E. coli K12 JA221/pIT141, NRRL-Hinterlegungs-Nr. B—15602) mit dem Restriktionsenzym MboII, Entfernen der erhaltenen Extensionen mit den Klenow-Fragment von DNA-Polymerase, Befestigen von BamHI-Linkern mit der Sequenz TGGATCCA und anschließend Ligation des likerhaltigen Fragments in BamHI-verdautes Plasmid pUC8. |

23. Transformante, enthaltend den rekombinanten DMA-Klonierungsvektor nach Anspruch 15, 18, 21 oder 22.

24. Transformante nach Anspruch 23, bei der es sich um E. coli handelt.

25. Transformante nach Anspruch 23, bei der es sich um Saccharomyces cereivisiae handelt.

26. Transformante, bei der es sich um eine E. coli-Wirtszelle mit einem Gehalt an dem Plamid nach Anspruch 20 handelt.

**Patentansprüche für den Vertragsstaat: AT**

1. Verfahren zur Herstellung eines Plasmids mit einer DNA-Sequenz, die für die letzten 338 Aminosäuren von Hygromycin B-phosphotransferase kodiert, entweder allein oder in translationaler Lesephase mit einem eine transkriptionale und translationale Aktivatorsequenz enthaltenden Gen oder einem Teil eines Gens, umfassend folgende Stufen: (a) Ligation des ~2,75 kb-SalI-BglII-Fragments des Plasmids pKC203 (ATCC Hinterlegungs-Nr 31912) und des ~4,1 kb-SalI-BglII-Fragments des Plasmid pKC7 (ATCC Hinterlegungs-Nr. 37084) unter Bildung des Plasmids pKC222; (b) Versehen des 325 bp-HphI-PstI-Fragments von Plasmid pKC222 mit einem BamHI-Linker und einem EcorRI-Linker; (c) Ligation des mit einem Linker versehenen Fragments von Stufe (b) mit dem EcorRI-BamHI-Verdauungsprodukt des bekannten Plasmids pBR322 zur Bildung des Plasmids pIT122; und (d) Ligation des ~1,45 kb EcoRI-Fragments von Plasmid pKC222 und des EcoRI-Verdauungsprodukts von Plasmid pIT122, unter Bildung des in Fig. 1 dargestellten Plasmids pIT123.

2. Verfahren nach Anspruch 1 zur Herstellung des Plasmids pIT144, umfassend die Ligation des ~1,3 kb-BamHI-BglII-Restriktionsfragments von Plasmid pIT123 nach Anspruch 1 in BamHI-verdautes Plasmid pUC7.

3. Verfahren nach Anspruch 1 zur Herstellung des Plasmids pIT212, umfassend die Ligation des ~1,3 kb-BamHI-BglII-Restriktionsfragments des Plasmid pIT123 nach Anspruch 1 in BAMHI-verdautes Plasmid pRB5 (ATCC-Hinterlegungs-Nr. 37051).

4. Verfahren nach Anspruch 3 zur Herstellung des Plasmids pIT213, umfassend die Ligation des ~1,7 kb-pVUII-Restriktionsfragments des Plasmids pNC59 (NRRL-Hinterlegungs-Nr. B—15604) in Smal-verdautes Plasmid pIT212 nach Anspruch 3.

5. Verfahren nach Anspruch 4 zur Herstellung des Plasmids pIT215, umfassend die Ligation des ~750 bp BamHI-BGlII-Restriktionsfragments des Plasmids pIT118 (NRRL-Hinterlegungs-Nr. B—15441) in BamHI-verdautes Plasmid pIT213 nach Anspruch 4.

6. Verfahren nach Anspruch 4 zur Herstellung des Plasmids pIT217, umfassend die Ligation des ~1 kb-BamHI-BglII-Restriktionsfragments von Plasmid pIT120 (NRRL-Hinterlegungs-Nr. B—15603) in BamHI-verdautes Plasmid pIT213 nach Anspruch 4 in der in Fig. 8 dargestellten Orientierung.

7. Verfahren nach Anspruch 1 zur Herstellung des Plasmids pKC307, umfassend die Ligation des HphI-verdauten Plasmids pIT104 und des HincII-verdauten Plasmids pUC8, wobei das Plasmid pIT104 konstruiert wird durch Versehen des SacI-geschnittenen Plasmids pKC222 nach Anspruch 1 mit BamHI-Linkern, Verdauung mit BamHI-Linkern, Verdauung mit BamHI-Restriktionsenzym und Ligation des erhaltenen Fragments mit dem EcoRI-BamHI-Verdauungsprodukt des bekannten Plasmids pBR322.

8. Verfahren nach Anspruch 7 zur Herstellung des Plasmids pKC308, umfassnd die Deletion des ~1,7 kb-HindIII-Fragments aus Plasmid pKC307.

9. Verfahren zur Herstellung des Plasmids pIT207, dargestellt in Fig. 3, umfassend die Ligation des ~750 bp-BamHI-BglII-Fragments des Plasmids pIT118 (NRRL-Hinterlegungs-Nr. B—15441) in BamHI-verdautes Plasmid pMC1587 (NRRL-Hinterlegungs-Nr. B—15442).

10. Verfahren nach Anspruch Herstellung des Plasmids pIT208, umfassend die Ligation des ~1,3 kb-BamHI-BglII-Restriktionsfragments von Plasmid pIT123 nach Anspruch 1 in BamHI-verdautes Plasmid pIT207 nach Anspruch 9.

11. Verfahren zur Herstellung des Plasmids pIT143, umfassend die Verdauung des 958 bp-ClaII-HincII-Fragments des Plasmids pIT141 (NRRL-Hinterlegungs-Nr. B—15602) mit dem Restriktionsenzym MboII, Entfernen der erhaltenen Extensionen, Anbringen von BamHI-Linkern und Ligation des linkerhaltigen Fragments in BamHI-verdautes Plasmid pUC8.

12. Verfahren nach Anspruch 4, zur Herstellung des Plasmids pIT219, umfassend die Ligation des ~230 bp-BamHI-Fragments des Plasmids pIT143 nach Anspruch 11 in BamHI-verdautes Plasmid pIT213 nach Anspruch 4.

13. Plasmid, hergestellt gemäß dem Verfahren von einem der Ansprüche 1 bis 12, vorliegend in einer E. coli-Wirtszelle.

14. Plasmid, hergestellt nach einem der Ansprüche 1 bis 12, vorliegend in einer Saccharomyces cerevisiae-Wirtszelle.

**Revendications pour les Etats contractants: BE CH DE FR GB IT LI LU NL SE**

1. ADN encodant les 338, 339 ou 340 derniers acides aminés de la phosphotransférase d'hygromycine B.

2. ADN selon la revendication 1, caractérisé en ce qu'il comprend:

```
Rm   R²n  CCT GAA CTC ACC GCG ACG TCT GTC GAG AAG
 ¦1  ¦3   ¦!¦ !!! !!! !!! !!! !!! !!! !!! !!! !!!
R¹m  R³n  GGA CTT GAG TGG CGC TGC AGA CAG CTC TTC

TTT CTG ATC GAA AAG TTC GAC AGC GTC TCC GAC CTG ATG
!!! !!! !!! !!! !!! !!! !!! !!! !!! !!! !!! !!! !!!
AAA GAC TAG CTT TTC AAG CTG TCG CAG AGG CTG GAC TAC

CAG CTC TCG GAG GGC GAA GAA TCT CGT GCT TTC AGC TTC
!!! !!! !!! !!! !!! !!! !!! !!! !!! !!! !!! !!! !!!
GTC GAG AGC CTC CCG CTT CTT AGA GCA CGA AAG TCG AAG

GAT GTA GGA GGG CGT GGA TAT GTC CTG CGG GTA AAT AGC
!!! !!! !!! !!! !!! !!! !!! !!! !!! !!! !!! !!! !!!
CTA CAT CCT CCC GCA CCT ATA CAG GAC GCC CAT TTA TCG

TGC GCC GAT GGT TTC TAC AAA GAT CGT TAT GTT TAT CGG
!!! !!! !!! !!! !!! !!! !!! !!! !!! !!! !!! !!! !!!
ACG CGG CTA CCA AAG ATG TTT CTA GCA ATA CAA ATA GCC

CAC TTT GCA TCG GCC GCG CTC CCG ATT CCG GAA GTG CTT
!!! !!! !!! !!! !!! !!! !!! !!! !!! !!! !!! !!! !!!
GTG AAA CGT AGC CGG CGC GAG GGC TAA GGC CTT CAC GAA

GAC ATT GGG GAA TTC AGC GAG AGC CTG ACC TAT TGC ATC
!!! !!! !!! !!! !!! !!! !!! !!! !!! !!! !!! !!! !!!
CTG TAA CCC CTT AAG TCG CTC TCG GAC TGG ATA ACG TAG

TCC CGC CGT GCA CAG GGT GTC ACG TTG CAA GAC CTG CCT
!!! !!! !!! !!! !!! !!! !!! !!! !!! !!! !!! !!! !!!
AGG GCG GCA CGT GTC CCA CAG TGC AAC GTT CTG GAC GGA

GAA ACC GAA CTG CCC GCT GTT CTG CAG CCG GTC GCG GAG
!!! !!! !!! !!! !!! !!! !!! !!! !!! !!! !!! !!! !!!
CTT TGG CTT GAC GGG CGA CAA GAC GTC GGC CAG CGC CTC

GCC ATG GAT GCG ATC GCT GCG GCC GAT CTT AGC CAG ACG
!!! !!! !!! !!! !!! !!! !!! !!! !!! !!! !!! !!! !!!
CGG TAC CTA CGC TAG CGA CGC CGG CTA GAA TCG GTC TGC

AGC GGG TTC GGC CCA TTC GGA CCG CAA GGA ATC GGT CAA
!!! !!! !!! !!! !!! !!! !!! !!! !!! !!! !!! !!! !!!
TCG CCC AAG CCG GGT AAG CCT GGC GTT CCT TAG CCA GTT

TAC ACT ACA TGG CGT GAT TTC ATA TGC GCG ATT GCT GAT
!!! !!! !!! !!! !!! !!! !!! !!! !!! !!! !!! !!! !!!
ATG TGA TGT ACC GCA CTA AAG TAT ACG CGC TAA CGA CTA

CCC CAT GTG TAT CAC TGG CAA ACT GTG ATG GAC GAC ACC
!!! !!! !!! !!! !!! !!! !!! !!! !!! !!! !!! !!! !!!
GGG GTA CAC ATA GTG ACC GTT TGA CAC TAC CTG CTG TGG

GTC AGT GCG TCC GTC GCG CAG GCT CTC GAT GAG CTG ATG
!!! !!! !!! !!! !!! !!! !!! !!! !!! !!! !!! !!! !!!
CAG TCA CGC AGG CAG CGC GTC CGA GAG CTA CTC GAC TAC
```

```
CTT TGG GCC GAG GAC TGC CCC GAA GTC CGG CAC CTC GTG
!!! !!! !!! !!! !!! !!! !!! !!! !!! !!! !!! !!! !!!
GAA ACC CGG CTC CTG ACG GGG CTT CAG GCC GTG GAG CAC

CAC GCG GAT TTC GGC TCC AAC AAT GTC CTG ACG GAC [AAC]
!!! !!! !!! !!! !!! !!! !!! !!! !!! !!! !!! !!! !!!
GTG CGC CTA AAG CCG AGG TTG TTA CAG GAC TGC CTG [TTG]

AAT GGC CGC ATA ACA GCG GTC ATT GAC TGG AGC GAG GCG
!!! !!! !!! !!! !!! !!! !!! !!! !!! !!! !!! !!! !!!
TTA CCG GCG TAT TGT CGC CAG TAA CTG ACC TCG CTC CGC

ATG TTC GGG GAT TCC CAA TAC GAG GTC GCC AAC ATC TTC
!!! !!! !!! !!! !!! !!! !!! !!! !!! !!! !!! !!! !!!
TAC AAG CCC CTA AGG GTT ATG CTC CAG CGG TTG TAG AAG

TTC TGG AGG CCG TGG TTG GCT TGT ATG GAG CAG CAG ACG
!!! !!! !!! !!! !!! !!! !!! !!! !!! !!! !!! !!! !!!
AAG ACC TCC GGC ACC AAC CGA ACA TAC CTC GTC GTC TGC

CGC TAC TTC GAG CGG AGG CAT CCG GAG CTT GCA GGA TCG
!!! !!! !!! !!! !!! !!! !!! !!! !!! !!! !!! !!! !!!
GCG ATG AAG CTC GCC TCC GTA GGC CTC GAA CGT CCT AGC

CCG CGG CTC CGG GCG TAT ATG CTC CGC ATT GGT CTT GAC
!!! !!! !!! !!! !!! !!! !!! !!! !!! !!! !!! !!! !!!
GGC GCC GAG GCC CGC ATA TAC GAG GCG TAA CCA GAA CTG

CAA CTC TAT CAG AGC TTG GTT GAC GGC AAT TTC GAT GAT
!!! !!! !!! !!! !!! !!! !!! !!! !!! !!! !!! !!! !!!
GTT GAG ATA GTC TCG AAC CAA CTG CCG TTA AAG CTA CTA

GCA GCT TGG GCG CAG GGT CGA TGC GAC GCA ATC GTC CGA
!!! !!! !!! !!! !!! !!! !!! !!! !!! !!! !!! !!! !!!
CGT CGA ACC CGC GTC CCA GCT ACG CTG CGT TAG CAG GCT

TCC GGA GCC GGG ACT GTC GGG CGT ACA CAA ATC GCC CGC
!!! !!! !!! !!! !!! !!! !!! !!! !!! !!! !!! !!! !!!
AGG CCT CGG CCC TGA CAG CCC GCA TGT GTT TAG CGG GCG

AGA AGC GCG GCC GTC TGG ACC GAT GGC TGT GTA GAA GTA
!!! !!! !!! !!! !!! !!! !!! !!! !!! !!! !!! !!! !!!
TCT TCG CGC CGG CAG ACC TGG CTA CCG ACA CAT CTT CAT

CTC GCC GAT AGT GGA AAC CGA CGC CCC AGC ACT CGT CCG
!!! !!! !!! !!! !!! !!! !!! !!! !!! !!! !!! !!! !!!
GAG CGG CTA TCA CCT TTG GCT GCG GGG TCG TGA GCA GGC

AGG GCA AAG GAA R⁴
!!! !!! !!! !!! !⁵
TCC CGT TTC CTT R
```

où

A représente le radical déoxyadényle,

G représente le radical déoxyguanidyle,

C représente le radical déoxycytidyle, et

T représente le radical thymidyle,

R et $R^2$ sont des triplets de déoxyribonucléotides qui encodent indépendamment la lysine,

$R^1$ et $R^3$ représentent des triplets de déoxyribonucléotids où les bases azotées sont complémentaires aux bases respectives et correspondantes de R et $R^2$, m et n = 0 ou 1, avec cette restriction que, lorsque n = 0, m = 0 et lorsque m = 1, n = 1,

$R^4$ représente un triplet de déoxyribonucléotide qui encode un codon d'arrêt de translation, et

$R^5$ représente un triplet de déoxyribonucléotide dans lequel les bases azotées sont complémentaires aux bases correspondantes de $R^4$,

avec cette restriction que l'ADN n'est pas associé à la séquence d'activateurs de transcription et de translation du plasmide pKC203 tel qu'il est obtenu à partir d'E. coli JR225 déposé sous le numéro matricule ATCC 31912.

3. ADN selon la revendication 2, caractérisé en ce qu'il est dans la phase de lecture de translation avec une séquence d'activateurs de transcription et de translation, avec cette restriction que la séquence n'est pas identique à celle encodée par le plasmide pKC203.

4. ADN selon la revendication 3, caractérisé en ce qu'il comprend jusqu'aux 15 premiers codons amino-terminaux d'un gène structural homologue à la séquence d'activateurs de transcription et de translation.

5. ADN selon la revendication 4, caractérisé en ce que $R^4$ représente TAG et $R^5$ représente ATC.

6. ADN selon l'une quelconque des revendications 1 à 5, caractérisé en ce que le gène homologue à la séquence d'activateurs de transcription et de translation est un gène bactérien.

7. ADN selon la revendication 6, caractérisé en ce que le gène encode jusqu'à 15 acides aminés.

8. ADN selon la revendication 6 ou 7, caractérisé en ce que le gène est une portion du gène E. coli lac Z.

9. ADN selon l'une quelconque des revendications 1 à 5, caractérisé en ce que le gène est un gène eucaryotique.

10. ADN selon la revendication 9, caractérisé en ce que le gène est une portion du gène de choc thermique de levure.

11. ADN selon l'une quelconque des revendications 2 à 10, caractérisé en ce que n = 1 et m = 0.

12. ADN selon l'une quelconque des revendications 2 à 10, caractérisé en ce que m et n = 1.

13. ADN selon l'une quelconque des revendications 2 à 10, caractérisé en ce que m et n = 0.

14. ADN selon la revendication 13, caractérisé en ce qu'il encode la séquence d'acides aminés:

```
          PRO GLU LEU THR ALA THR SER VAL GLU LYS

      PHE LEU ILE GLU LYS PHE ASP SER VAL SER ASP LEU MET

      GLN LEU SER GLU GLY GLU GLU SER ARG ALA PHE SER PHE

      ASP VAL GLY GLY ARG GLY TYR VAL LEU ARG VAL ASN SER

      CYS ALA ASP GLY PHE TYR LYS ASP ARG TYR VAL TYR ARG

      HIS PHE ALA SER ALA ALA LEU PRO ILE PRO GLU VAL LEU

      ASP ILE GLY GLU PHE SER GLU SER LEU THR TYR CYS ILE

      SER ARG ARG ALA GLN GLY VAL THR LEU GLN ASP LEU PRO

      GLU THR GLU LEU PRO ALA VAL LEU GLN PRO VAL ALA GLU

      ALA MET ASP ALA ILE ALA ALA ALA ASP LEU SER GLN THR

      SER GLY PHE GLY PRO PHE GLY PRO GLN GLY ILE GLY GLN

      TYR THR THR TRP ARG ASP PHE ILE CYS ALA ILE ALA ASP

      PRO HIS VAL TYR HIS TRP GLN THR VAL MET ASP ASP THR

      VAL SER ALA SER VAL ALA GLN ALA LEU ASP GLU LEU MET

      LEU TRP ALA GLU ASP CYS PRO GLU VAL ARG HIS LEU VAL

      HIS ALA ASP PHE GLY SER ASN ASN VAL LEU THR ASP ASN

      GLY ARG ILE THR ALA VAL ILE ASP TRP SER GLU ALA MET

      PHE GLY ASP SER GLN TYR GLU VAL ALA ASN ILE PHE PHE

      TRP ARG PRO TRP LEU ALA CYS MET GLU GLN GLN THR ARG

      TYR PHE GLU ARG ARG HIS PRO GLU LEU ALA GLY SER PRO

      ARG LEU ARG ALA TYR MET LEU ARG ILE GLY LEU ASP GLN
```

```
LEU TYR GLN SER LEU VAL ASP GLY ASN PHE ASP ASP ALA

ALA TRP ALA GLN GLY ARG CYS ASP ALA ILE VAL ARG SER

GLY ALA GLY THR VAL GLY ARG THR GLN ILE ALA ARG ARG

SER ALA ALA VAL TRP THR ASP GLY CYS VAL GLU VAL LEU

ALA ASP SER GLY ASN ARG ARG PRO SER THR ARG PRO ARG

ALA LYS GLU
```

où

MET représente la méthionine,
LYS représente la lysine,
PRO représente la proline,
GLU représente l'acide glutamique,
LEU représente la leucine
THR représente la thréonine,
ALA représente l'alanine,
SER représente la sérine,
VAL représente la valine,
PHE représente la phénylalanine,
ILE représente l'isoleucine,
GLY représente la glycine,
ASP représente l'acide aspartique,
GLN représente la glutamine,
ARG représente l'arginine,
CYS représente la cystéine,
TRP représente la tryptophane,
ASN représente l'asparagine,
HIS représente l'histidine, et
TYR représente la tyrosine.

15. Plasmide pIT123 représenté en figure 1 et construit par les étapes consistant à: (a) ligaturer un fragment à ~2,75 kb de SalI-BglII du plasmide pKC203 (numéro de dépôt ATCC 31912) et le fragment à ~4,1 kb de SalI-BglII du plasmide PKC7 (numéro de dépôt ATCC 37084) pour former le plasmide pKC222; (b) fournir, au fragment à 325 bp de HphI-PstI du plasmide pKC222 d'un agent de liaison de BamHI et d'un agent de liaison EcoRI; (c) ligaturer le fragment pourvu de l'agent de liaison de l'étape (b) avec le produit de digestion EcoRI-BamHI du plasmide connu pBR322 pour obtenir le plasmide pIT122; et ligaturer le fragment à ~1,45 kb d'EcorRI du plasmide pKC222 et le produit de digestion d'EcoRI du plasmide pIT122.

16. ADN selon la revendication 2, caractérisé en ce qu'il est le fragment de restriction à ~1,3 kb de BamHI-BglII du plasmide pIT123 selon la revendication 15.

17. Vecteur de clonage d'ADN recombinant comprenant l'ADN selon l'une quelconque des revendications 1 à 14 ou le revendication 16.

18. Vecteur selon la revendication 17, caractérisé en ce qu'il est un plasmide.

19. Plasmide pIT144 qui est construit en ligaturant le fragment à ~1,3 kb de BamHI-BglII du plasmide pIT123 selon la revendication 15, et le produit de digestion par BamHI du plasmide connu pUC7.

20. Plasmide pIT207 qui est illustré en figure 3 et qui est construit en ligaturant le fragment à ~750 bp de BamHI-BglII du plasmide pIT118 (numéro de dépôt NRRL B—15441) et le produit de digestion de BamHI à 16 kb du plasmide pMC1587 (numéro de dépôt NRRL B—15442).

21. Plasmide pIT208 qui est illustré en figure 3 et qui est construit en ligaturant le fragment à ~1,3 kb de BamHI-BglII du plasmide pIT123 selon la revendication 15, et le produit de digestion par BamHI du plasmide pIT207 selon la revendication 20.

22. Vecteur selon la revendication 18, à savoir:

| Plasmide | Dimension (en kb) | Construit par |
|---|---|---|
| pIT212 | 8,9 | Ligature du fragment à ~1,3 kb de BamII-BglII du plasmide pIT123 selon la revendication 15 en un site unique de restriction de BamHI du plasmide pRB5 (numéro de dépôt ATCC 37051). |
| pIT213 | 10,6 | Ligature des extrémités émoussées du fragment à ~1,7 kb de PvuII du plasmide pNG59* (numéro de dépôt NRRL B—15604) en site unique de restriction SamI du plasmide pIT212. |
| pIT215 | 11,4 | Ligature du fragment à ~750 bp de BamHI-BglII du plasmide pIT118 (numéro de dépôt NRRL B—15441) en site unique de restriction BamHI du plasmide pIT213 dans l'orientation illustrée en figure 8. |
| pIT217 | 11,7 | Ligature du fragment à ~1 kb de BamHI-BglII du plasmide pIT120 (numéro de dépôt NRRL B—15603) en site unique de restriction BamHI du plasmide pIT213 dans l'orientation illustrée en figure 8. |
| pIT219 | 10,8 | Ligature du fragment à ~230 bp de BamHI du plasmide pIT143 en site unique de restriction BamHI du plasmide pIT213 dans l'orientation illustrée en figure 9, ce plasmide pIT143 étant construit en mettant à digérer le fragment de ClaI-HincII à 958 bp du plasmide pIT141 (isolé de manière conventionnelle de E. coli K12 JA221/pIT141, numéro de dépôt NRRL B—15602) avec l'enzyme de restriction MBoII en éliminant les extensions obtenues avec le fragment de Klenow de la polymérase d'ADN, en fixant les agents de liaison BamHI avec la séquence TGGATCCA, puis en ligaturant le fragment contenant les agents de liaison dans le plasmide pUC8 mis à digérer avec BamHI. |

23. Transformant comprenant le vecteur de clonage d'ADN recombinant selon la revendication 15, 18, 21 ou 22.

24. Transformant selon la revendiction 23, caractérisé en ce qu'il est E. coli.

25. Transformant selon la revendication 23, caractérisé en ce qu'il est Saccharomyces cerevisiae.

26. Transformant, caractérisé en ce qu'il est une cellule hôte d'E. coli comprenant le plasmide selon la revendication 20.

## Revendications pour l'Etat contractant: AT

1. Procédé de préparation d'un plasmide comprenant une séquence d'ADN encodant les 338 derniers acides aminés de la phosphotransférase d'hygromycine B, seul ou en phase de lecture de translation avec un gène ou une portion de gène contenant une séquence d'activateurs de transcription et de translation, caractérisé en ce qu'il comprend les étapes qui consistent à: (a) ligaturer le fragment à ~2,75 kb de SalI-BglII du plasmide pKC203 (numéro de dépôt ATCC 31912) et le fragment à ~4,1 kb de SalI-BglII du plasmide pKC7 (numéro de dépôt ATCC 37084) pour former le plasmide pKC222; (b) fournir, au fragment à 325 bp de HphI-PstI du plasmide pKC222, un agent de liaison BamHI et un agent de liaison EcoRI; (c) ligaturer le fragment pourvu de la liaison de l'étape (b) avec le produit de digestion par EcoRI-BamHI du plasmide connu pBR322 pour obtenir le plasmide pIT122; et (d) ligaturer le fragment à ~1,45 kb d'EcoRI du plasmide pKC222 et le produit de digestion EcoRI du plamide pIT122 pour obtenir le plasmide pIT123 illustré en figure 1.

2. Procédé selon la revendication 1, en vue de préparer le plasmide pIT144, caractérisé en ce qu'il consiste à ligaturer le fragment de restriction à ~1,3 kb de BamHI-BglII du plasmide pIT123 de la revendication 1 en un plasmide pUC7 mis à digérer avec BamHI.

3. Procédé selon la revendication 1, en vue de préparer le plasmide pIT212, caractérisé en ce qu'il consiste à ligaturer le fragment de restriction à environ 1,3 kb de BamHi-BglII du plasmide pIT123 de la revendication 1 en plasmide pRB5 mis à digérer avec BamHI (numéro de dépôt ATCC 37051).

4. Procédé selon la revendication 3, en vue de préparer le plasmide pIT213, caractérisé en ce qu'il consiste à ligaturer le fragment de restriction de PvuII à ~1,7 kb du plasmide pNC59* (numéro de dépôt NRRL B—15604) en plasmide pIT212 mis à digérer avec SmaI selon la revendication 3.

5. Procédé selon la revendication 4, en vue de préparer le plasmide pIT215, caractérisé en ce qu'il consiste à ligaturer le fragment de restriction de BamHI-BGIII à ~750 bp du plasmide pIT118 (numéro de dépôt NRRL B—15441) en plasmide pIT213 mis à digérer avec BamHI selon la revendication 4.

6. Procédé selon la revendication 4 pour la préparation du plasmide pIT217, caractérisé en ce qu'il consiste à ligaturer le fragment de restriction de BamHI-BglII à ~1 kb du plasmide pIT120 (numéro de dépôt NRRL B—15603) en un plasmide pIT213 mis à digérer avec BamHI selon la revendication 4 dans l'orientation illustrée en figure 8.

7. Procédé selon la revendication 1 pour la préparation du plasmide pKC307, caractérisé en ce qu'il consiste à ligaturer le plasmide pIT104 mis à digérer avec HphI et le plasmide pUC8 mis à digérer avec HincII, le plasmide pIT104 étant construit en fournissant, au plasmide pKC222 de la revendication 1 incisé par SacI, avec des agents de liaison BamHI, mettre à digérer avec l'enzyme de restriction BamHI et ligaturer le fragment obtenu avec le produit de digestion EcoRI-BamHI du plasmide connu bpBR322.

8. Procédé selon la revendication 7, pour la préparation du plasmide pKC308, caractérisé en ce qu'il consiste à supprimer le fragment de HindIII à ~1,7 kb hors du plasmide pKC307.

9. Procédé de prépartion du plasmide pIT207 illustré en figure 3, caractérisé en ce qu'il consiste à ligaturer le fragment à ~750 bp de BamHI-BglII du plasmide pIT118 (numéro de dépôt NRRL B—15441) en plasmide pMC1587 mis à digérer avec BamHI (numéro de dépôt NRRI B—15442).

10. Procédé selon la revendication 1, pour la préparation du plasmide pIT208, caractérisé en ce qu'il consiste à ligaturer le fragment de restriction de BamHI-BglII à ~1,3 kb du plasmide pIT123 selon la revendication 1 en plasmide pIT207 mis à digérer avec BamHI selon la revendication 9.

11. Procédé de préparation du plasmide pIT143, caractérisé en ce qu'il consiste à mettre à digérer le fragment de ClaI-HincII à 958 bp du plasmide pIT141 (numéro de dépôt NRRL B—15602) avec l'enzyme de restriction MboII, éliminer les extensions obtenues fixant les agents de liaison de BamHI et ligaturer le fragment contenant les agents de liaison dans le plasmide pUC8 mis à digérer avec BamHI.

12. Procédé selon la revendication 4 pour la préparation du plasmide pIT219, caractérisé en ce qu'il consiste à ligaturer le fragment à 230 bp de BamHI du plasmide pIT143 selon la revendication 11 en plasmide pIT213 selon la revendication 4, mis à digérer avec BamHI.

13. Plasmide préparé selon le procédé de l'une quelconque des revendication 1 à 12, présent dans une cellule hôte d'E. coli.

14. Plasmide préparé selon l'une quelconque des revendications 1 à 12, présent dans une cellule hôte de Saccharomyces cerevisiae.

# FIG. I
## Restriction Site Map of Plasmids
## pIT123 and pKC222

pIT123

pKC222

# FIG. 2
## Restriction Site Map of Plasmids pKC203 and pIT144

pKC203

pIT144

# FIG. 3
## Restriction Site Map of Plasmids
## pIT208 and pIT207

pIT208

pIT207

## FIG. 4
## Restriction Site Map of Plasmids
## pKC307 and pIT125

pKC307

pIT125

# FIG. 5
## Thymosin Alpha I Gene

```
    1    2   3   4   5   6   7   8   9  10  11  12  13  14   15  16  17  18  19  20  21  22  23  24  25  26  27  28
   Met  Ser Asp Ala Ala Val Asp Thr Ser Ser Glu Ile Thr Thr Lys Asp Leu Lys Glu Lys Lys Glu Val Val Glu Glu Ala Glu Asn
```

Eco RI

stop stop

←——T₁——→    ←———T₂———→    ←———T₃——→    ←——T₄——→   ←——T₉——→    ←———T₁₀———→   ←——T₁₁——→   ←——T₁₂——→

AATTCATGTCTGATGCTGCTGTTGATACTTCTTCTGAGATTACTACTAAAGATCTTAAGGAGAAGAAGGAAGTTGTCGAAGAGGCTGAGAACTAATAG
GTACAGACTACGACGACAACTATGAAGAAGACTCTAATGATGATTTCTAGAATTCCTCTTCTTCCTTCAACAGCTTCTCCGACTCTTGATTATCCTAG

←———T₅———→   ←——T₆——→   ←——T₇——→   ←——T₈——→   ←——T₁₃——→   ←——T₁₄——→   ←——T₁₅——→   ←—T₁₆——→

Bgl II                                                                          Bam HI

# FIG. 6
## Synthesis Procedure for Fragment T₁₅

# FIG. 7
# Construction Route for Plasmid pTh α 1

# FIG. 8
## Restriction Site Map of Plasmids
## pIT215 and pIT217

pIT215

pIT217

# FIG. 9
## Restriction Site Map of Plasmid pIT219

pIT219